(19) Europäisches Patentamt
European Patent Office
Office européen des brevets

(11) Publication number: **0 370 712 A2**

(12) **EUROPEAN PATENT APPLICATION**

(21) Application number: **89311961.0**

(22) Date of filing: **17.11.89**

(51) Int. Cl.$^5$: **C07D 211/32, C07D 211/22, C07D 295/15, C07D 211/70, C07C 255/24, C07D 401/06, C07D 409/06, C07D 405/06, A61K 31/445**

(30) Priority: **18.11.88 JP 293408/88**
**30.11.88 JP 303461/88**
**03.02.89 JP 26232/89**
**16.03.89 JP 64059/89**

(43) Date of publication of application:
**30.05.90 Bulletin 90/22**

(84) Designated Contracting States:
**CH DE FR GB IT LI**

(71) Applicant: **AJINOMOTO CO., INC.**
**5-8, Kyobashi 1-chome, Chuo-ku**
**Tokyo 104(JP)**

(72) Inventor: **Syoji, Masataka Central Research Laboratories**
**Ajinomoto Co., Inc. No 1-1, Suzuki-cho**
**Kawasaki-ku**
**Kawasaki-shi Kanagawa-ken(JP)**
Inventor: **Toyota, Kozo Central Research Laboratories**
**Ajinomoto Co., Inc. No 1-1, Suzuki-cho**
**Kawasaki-ku**
**Kawasaki-shi Kanagawa-ken(JP)**
Inventor: **Eguchi, Chikahiko Central Research Laboratories**
**Ajincmoto Co., Inc. No 1-1, Suzuki-cho**
**Kawasaki-ku**
**Kawasaki-shi Kanagawa-ken(JP)**
Inventor: **Domoto, Hideki Central Research Laboratories**
**Ajinomoto Co., Inc. No 1-1, Suzuki-cho**
**Kawasaki-ku**
**Kawasaki-shi Kanagawa-ken(JP)**
Inventor: **Yoshimoto, Ryota Central Research Laboratories**
**Ajinomoto Co., Inc. No 1-1, Suzuki-cho**
**Kawasaki-ku**
**Kawasaki-shi Kanagawa-ken(JP)**
Inventor: **Kamimura, Akira Central Research Laboratories**
**Ajinomoto Co., Inc. No 1-1, Suzuki-cho**
**Kawasaki-ku**
**Kawasaki-shi Kanagawa-ken(JP)**

(74) Representative: **Harrison, David Christopher et al**
**MEWBURN ELLIS 2 Cursitor Street**
**London EC4A 1BQ(GB)**

# (54) Ethylamine derivatives and hypotensives containing the same.

(57) An ethylamine derivative of general formula (I) or a pharmaceutically acceptable salt thereof;

$$Q-X-CH_2CH_2-\overset{Z}{\overset{|}{N}}-CH_2CH_2-\overset{W}{\overset{|}{Y}} \qquad (I)$$

wherein:
X represents an optionally substituted alkylene bridge, of which one or more carbon atoms may be substituted with an optionally substituted hetero atom and which may include an unsaturated bond;
Y represents any one of several specified aryl-containing groups;
Z represents a methyl group or in combination with W represents an ethylene group ($-CH_2CH_2-$);
W represents a hydrogen atom in the case that W is not combined with Z; and
Q represents any one of several specified aryl-containing groups.
The compounds are useful as hypotensives.

EP 0 370 712 A2

## ETHYLAMINE DERIVATIVES AND HYPOTENSIVES CONTAINING THE SAME

The present invention relates to novel ethylamine derivatives and hypotensives containing the same.

It is said that there are about 13,000,000 patients with hypertension in Japan and its frequency of occurrence becomes higher as age is advanced. Further, hypertension has provided an important background, for cerebral apoplexy and heart diseases rank second and third in causes of death. Accordingly, among drugs for treating adult diseases, a hypotensive is considered to be one of the most important drugs.

In patients with hypertension, more than 90% suffer from essential hypertension, a cause of which is not exactly known, and its treatment is merely within symptomatic therapy. Therefore, it is necessary for patients to be administered with hypotensives throughout their lifetime. For this reason, high safety, sure effects and durability of the effects are required for hypotensives.

Accordingly, it is required to develop hypotensives having excellent activity and which can be industrially prepared at low cost in a simple manner.

As a result of extensive investigations to solve the problems described above, the present inventors have found that novel ethylamine derivatives represented by general formula (I):

$$Q - X - CH_2CH_2 - \overset{\overset{\displaystyle Z}{|}}{N} - CH_2CH_2 - \overset{\overset{\displaystyle W}{|}}{Y} \qquad \text{(I)}$$

have excellent hypotensive activity, its synthesis steps are simple and the ethylamine derivatives can be easily prepared industrially at low cost.

In general formula (I), Y represents any one of the following :

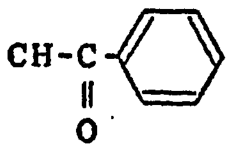 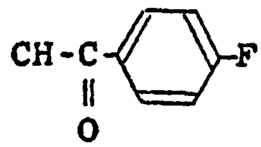 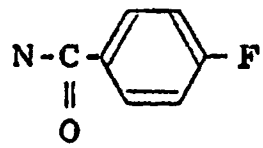

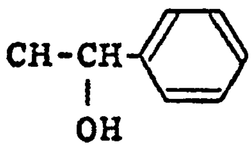 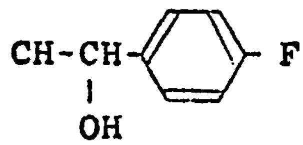

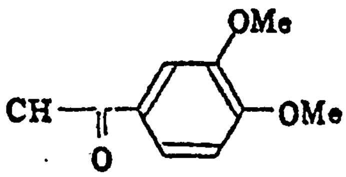 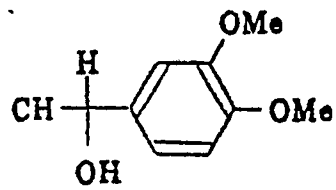

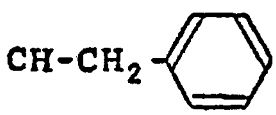 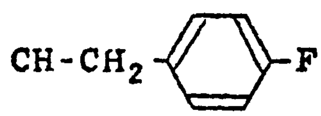

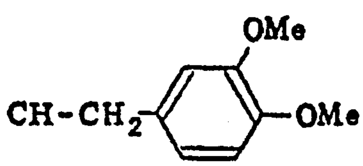 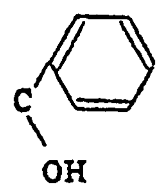

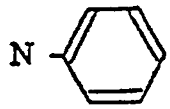

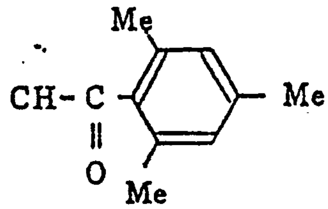

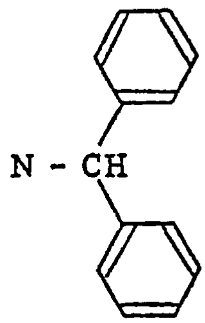

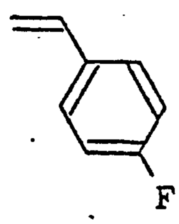

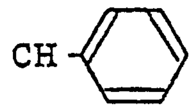

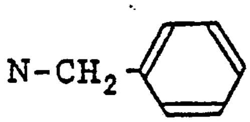

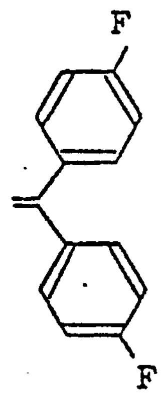

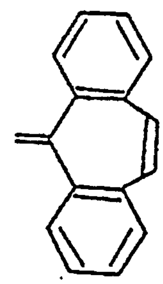

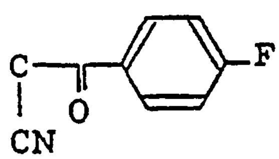

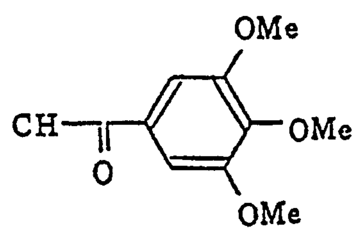

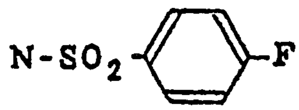

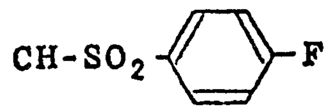

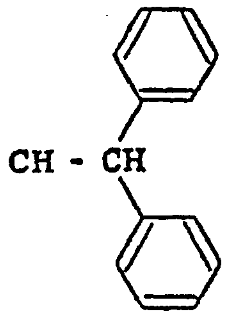

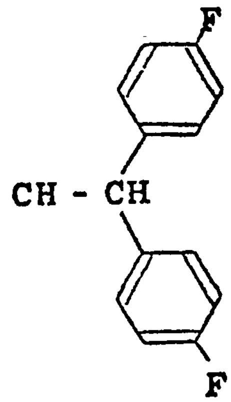

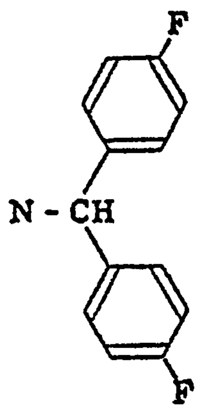

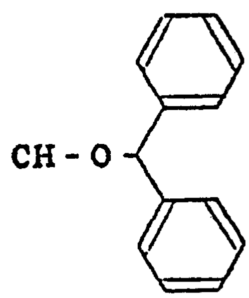

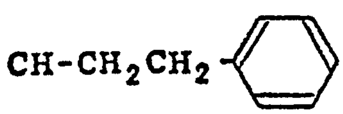

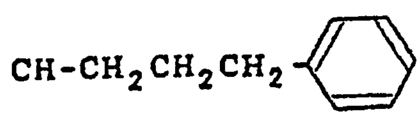

X represents an optionally substituted alkylene bridge, of which one or more carbon atoms may be substituted with an optionally substituted hetero atom and which may include an unsaturated bond;
Z represents a methyl group or in combination with W represents an ethylene group ($-CH_2CH_2-$);
W represents a hydrogen atom in the case that W is not combined with Z;
Q represents any one of the following:

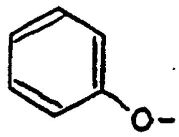

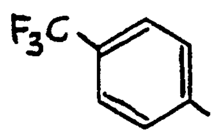

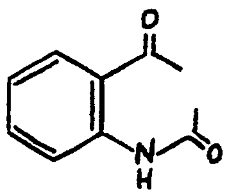

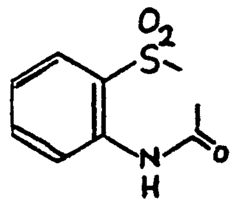

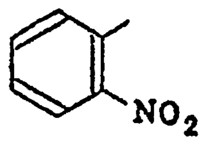

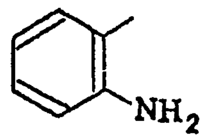

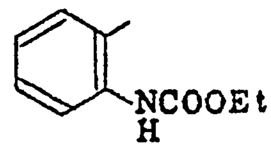

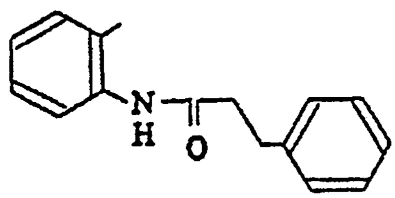

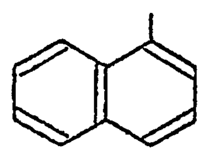

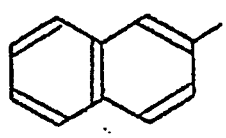

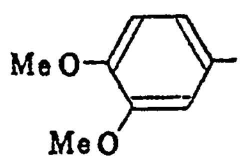

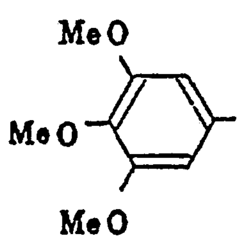

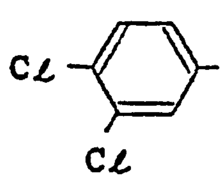

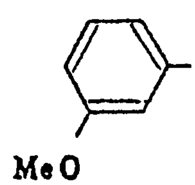

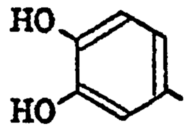

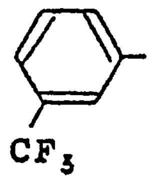

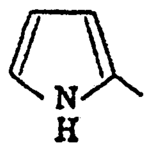

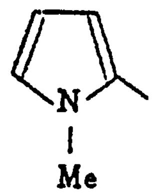

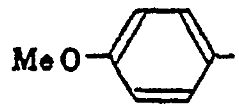

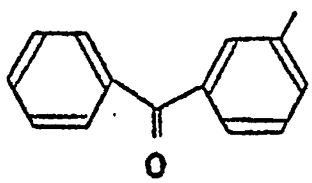

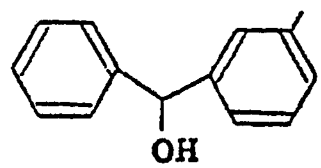

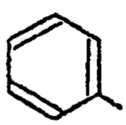

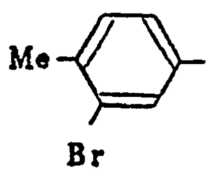

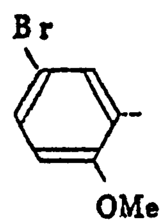

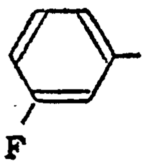

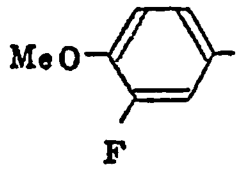

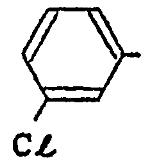

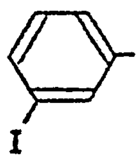

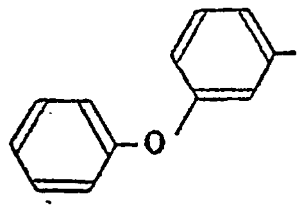

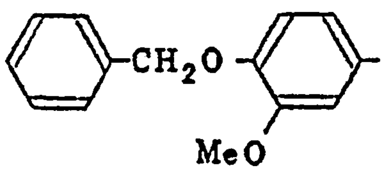

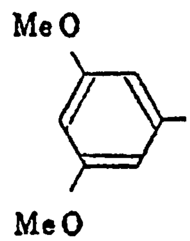

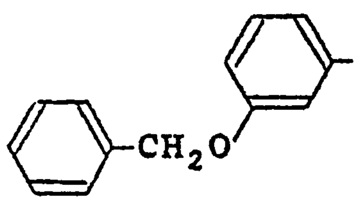

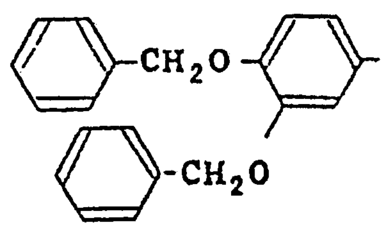

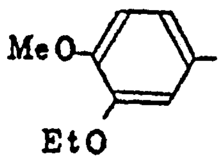

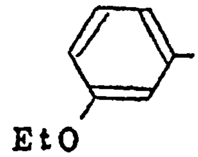

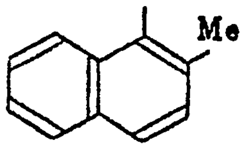

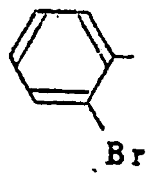

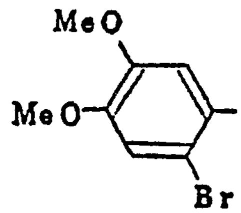

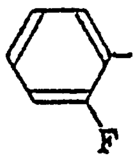

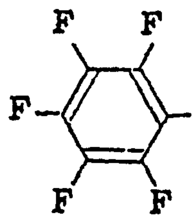

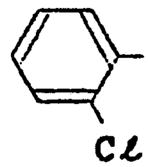

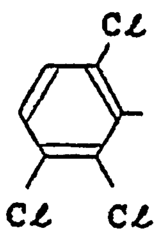

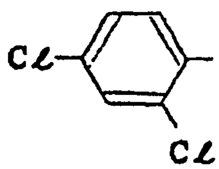

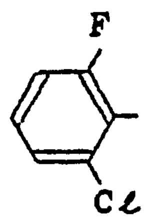

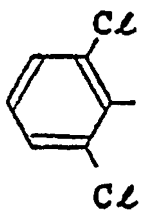

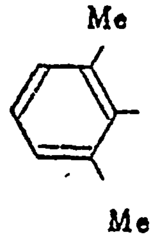

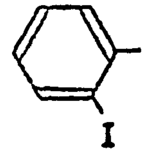

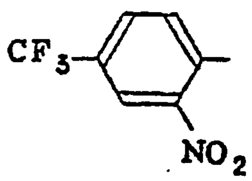

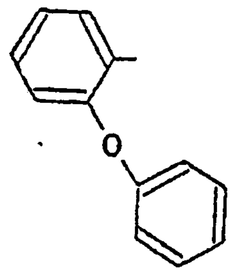

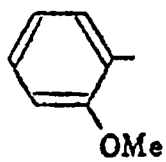

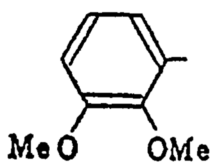

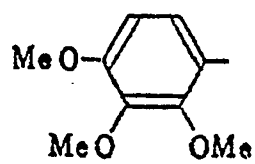

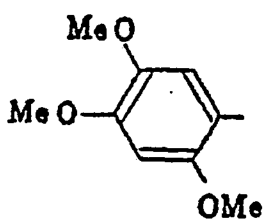

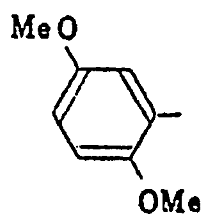

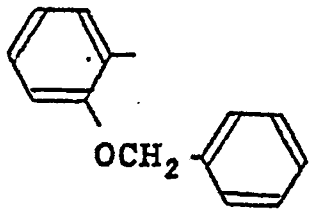

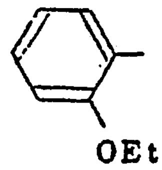

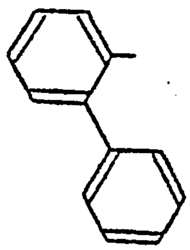

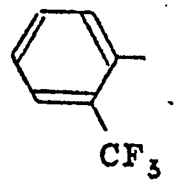

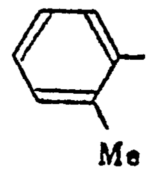

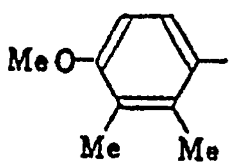

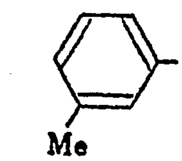

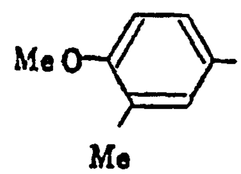

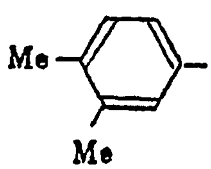

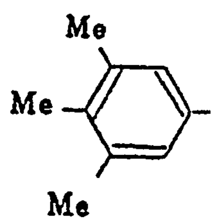

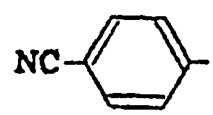

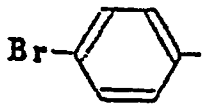

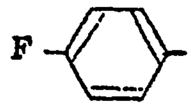

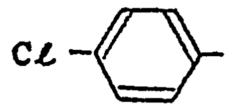

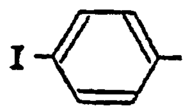

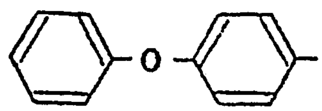

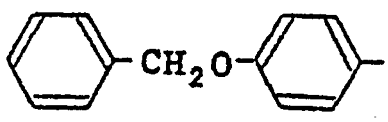

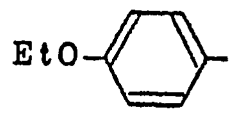

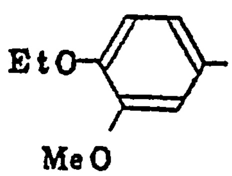

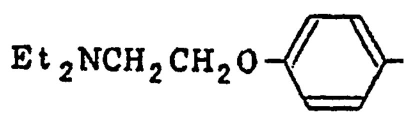

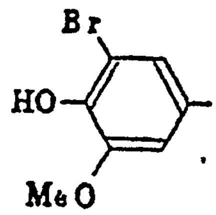
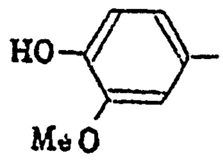
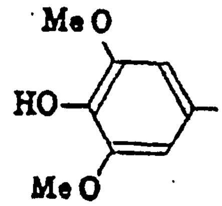

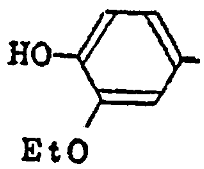
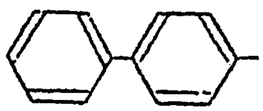
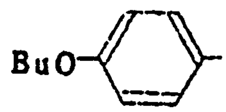

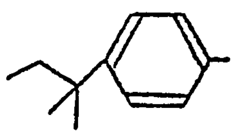
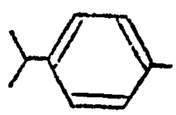
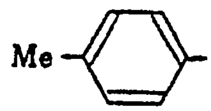

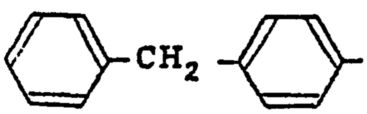
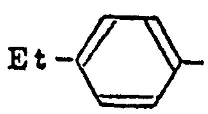
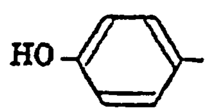

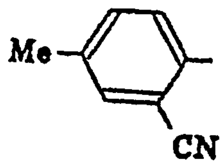
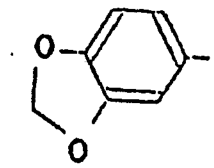
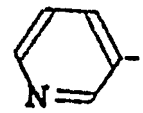

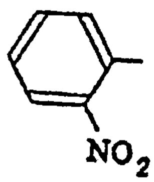
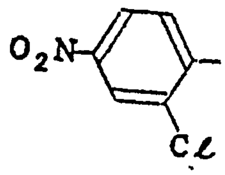
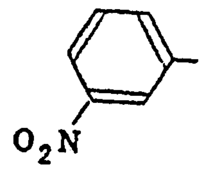

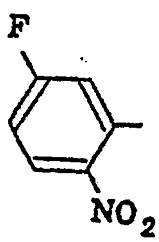
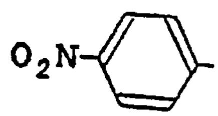
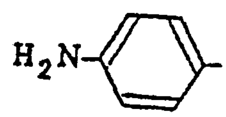

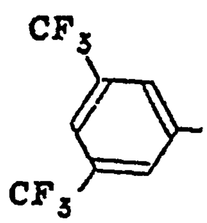
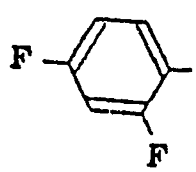
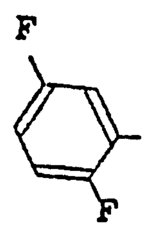

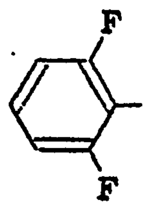

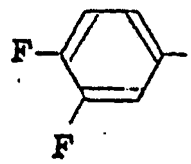

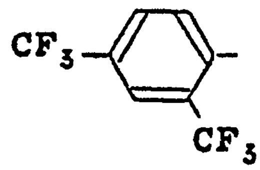

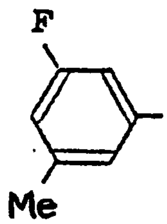

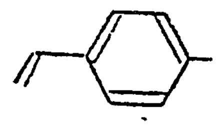

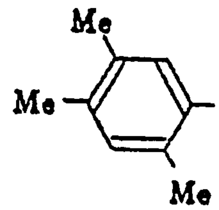

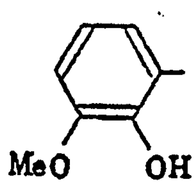

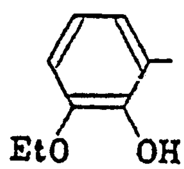

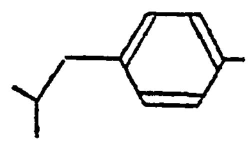

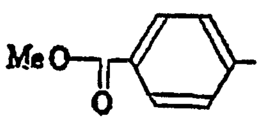

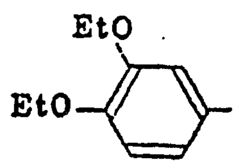

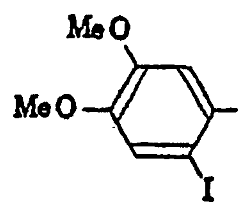

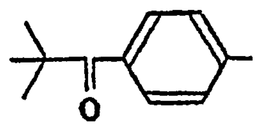

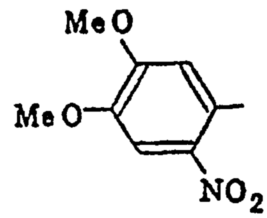

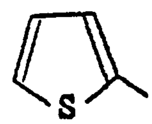

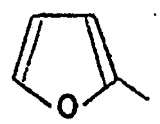

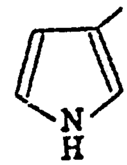

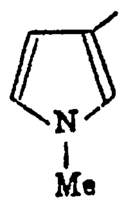

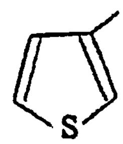

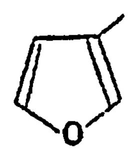

The ethylamine derivatives of the present invention may be in the form of salts thereof.

The ethylamine derivatives of the present invention are useful as hypotensives for treating mammals including human who suffer from hypertension. The ethylamine derivatives are utilizable for reducing blood pressure in the form of preparations such as tablets, capsules or elixir for oral administration and for parenteral administration, by formulation into a solution in a sterile solvent or a suspension. The ethylamine derivatives of the present invention can be administered to patients (animal and human) who require such treatment, in a dose of 0.2 to 500 mg generally in several portions; therefore, in a daily dose of 1 to 2000 mg. Dose may be varied depending upon degree of severity of disease, body weight of the patient and other factors recognized by one skilled in the art.

Further the ethylamine derivatives of the present invention may be used in combination with other hypotensives. Examples of such hypotensives usable in combination include $\alpha_1$-antagonists such as Prazosin, etc.; calcium antagonists such as Nifedipine, Nicardipine, Diltiazem, Verapamil, etc.; convertase inhibitors such as Captopril, Enarapril, etc.

Typical combination use shown above comprises formulation into pharmaceutical compositions as mentioned below. Approximately 0.2 to 500 mg of the derivative of the present invention or a physiologically acceptable salt compound or a mixture thereof is mixed with physiologically acceptable vehicles, carriers, exipients, binders, preservatives, stabilizers, flavors, etc., in generally acceptable unit dose forms required for preparation of drugs. A quantity of the active substance in these compositions or pharmaceutical compositions is controlled so as to give an appropriate dose within a given range.

Specific examples of drugs which can be mixed into tablets capsules, etc. include the following: binders such as tragacanth, gum arabic, corn starch or gelatin; excipients such as microcrystalline cellulose; swelling agents such as corn starch, pregelatinated starch or alginic acid; sweeteners such as sucrose, lactose or saccharine; flavors (e.g., peppermint, Gaultheria adenothrix oil or cherry. In case that the unit for pharmaceutical preparations takes a capsule form, liquid carriers such as oils and fats may also further be contained. Various other materials may be present as coating agents or in order to change physical mode of unit for pharmaceutical preparations in another method. For example, tablets may be coated either with shellac or sugar or with both. Syrups or elixir contains the active compound and may contain sucrose as a sweetener, methyl and propyl paraven as preservativės, dyes and flavors such as cherry or orange flavor.

Sterile compositions for injection may be prepared in a conventional pharmaceutical manner by dissolving or suspending the active substance in a vehicle such as water for injection, or naturally occurring vegetable oils such as sesame oil, palm oil, peanut oil, cotton seed oil, etc. or synthetic fat vehicle such as

ethyl oleate, etc. Buffer, preservatives, antioxidants, etc. may also be used, if necessary.

## Examples

Hereafter the present invention will be described in detail with reference to the examples below.

Unless otherwise indicated, developing conditions for TLC were chloroform/methanol = 9/1; mass spectrum (MS) was performed in FD mode (m/z) and nuclear magnetic resonance spectrum (NMR) was measured using tetramethylsilane as the internal standard and deuterium chloroform as the solvent.

### Example 1 (illustrative)

2-(3,4-Dimethoxyphenyl)-2-dodecyl-5-(4-(4-fluorobenzoyl)-1-piperidinyl)valeronitrile hydrochloride

This Example illustrates the experimental procedure used in the preparation of the compounds of Examples 1 to 50 which follow.

a) In a nitrogen atmosphere, 50 ml of a solution of 17.7 g (100 mmols) of 3,4-dimethoxyphenylacetonitrile in 50 ml of 1,2-dimethoxyethane (DME) was dropwise added to a suspension of 4.7 g (120 mmols) of sodium amide in 80 ml of DME at room temperature. After completion of the dropwise addition, the reaction mixture was stirred at 50°C for an hour. After allowing to cool, a solution of 24.9 g (100 mmols) of 1-bromododecane in 20 ml of DME was dropwise added. After completion of the dropwise addition, the reaction mixture was stirred at room temperature for an hour and at 50°C for 2 hours. After again allowing to cool, 4.7 g (120 mmols) of sodium amide was added thereto followed by stirring at 50°C for 2 hours. After allowing to cool, a solution of 15.7 g (100 mmols) of 1-bromo-3-chloro-propane in 20 ml of DME was dropwise added. After completion of the dropwise addition, the reaction mixture was stirred at room temperature for an hour and at 50°C for 2 hours. After allowing to cool, water was added to the reaction mixture. The mixture was extracted with ethyl acetate. After drying over anhydrous magnesium sulfate, the solvent was distilled off under reduced pressure. The obtained residue was purified by silica gel column chromatography (hexane/ethyl acetate = 6 : 1) to give the title compound.

Amount yielded 15.0 g (35.6 mmols)
Yield 35.6%
TLC (chloroform) Rf = 0.51
MS 421 (M+)
NMR
0.80 (3H, t), 1.1-2.2 (26H, m), 3.47 (2H, t), 3.85 (3H, s), 3.88 (3H, s), 6.9-7.0 (3H, m)

b) 2-(3,4-Dimethoxyphenyl)-2-dodecyl-5-(4-(4-fluorobenzoyl)-1-piperidinyl)valeronitrile hydrochloride

A suspension of 2.1 g (5.0 mmols) of 5-chloro-2-(3,4-dimethoxyphenyl)-2-dodecyl-5-(4-(4-fluorobenzoyl)-1-piperidinyl)-valeronitrile hydrochloride, 1.2 g (5.0 mmols) of 4-(4-fluorobenzoyl)piperidine hydrochloride, 2.0 g (15 mmols) of potassium carbonate and 2.2 g (15 mmols) of sodium iodide in 50 ml of methyl isobutyl ketone was reacted under reflux overnight. After completion of the reaction, water was added to the reaction mixture. The mixture was extracted with ethyl acetate. After drying over anhydrous magnesium sulfate, the solvent was distilled off under reduced pressure. The obtained residue was purified by silica gel column chromatography (chloroform) to give a free base of the title compound. Then, the base was converted into the hydrochloride using an equimolar amount of 4 normal dioxane solution of hydrogen chloride.

Amount yielded 0.98 g (1.56 mmol)
Yield 31%
TLC Rf = 0.70
MS 592 (M+)
NMR
0.84 (3H, t), 1.2-1.5 (22H, m), 1.8-2.1 (8H, m), 2.9-3.1 (4H, m), 3.4-3.5 (2H, m), 3.6-3.8 (1H, m), 3.76 (3H, s), 3.79 (3H, s), 6.9-7.0 (3H, m), 7.40 (2H, dd)

Example 2

2-(3,4-Dimethoxyphenyl)-5-(4-(α-hydroxy-4-fluorobenzyl)-1-piperidinyl)-2-tridecylvaleronitrile hydrochloride

TLC Rf = 0.38
MS 608 (M+)
NMR
0.88 (3H, t), 1.1-1.3 (24H, m), 1.8-3.6 (16H, m), 3.8-3.9 (6H, m), 4.41 (1H, d), 6.8-7.3 (7H, m)

Example 3

2-(3,4-Dimethoxyphenyl)-5-(4-(α-hydroxy-4-fluorobenzyl)-1-piperidinyl)-2-tetradecylvaleronitrile hydrochloride

TLC Rf = 0.40
MS 622 (M+)
NMR
0.87 (3H, t), 1.1-1.3 (26H, m), 1.8-3.6 (16H, m), 3.8-3.9 (6H, m), 4.41 (1H, d), 6.8-7.3 (7H, m)

Example 4

2-Decyl-2-(3,4-dimethoxyphenyl)-6-(4-(4-fluorobenzoyl)-1-piperidinyl)hexanenitrile hydrochloride

TLC Rf = 0.71
MS 578 (M+)
NMR
0.84 (3H, t), 1.2-1.5 (18H, m), 1.6-2.1 (10H, m), 2.9-3.1 (4H, m), 3.4-3.5 (2H, m), 3.6-3.8 (1H, m), 3.77 (3H, s), 3.79 (3H, s), 6.9-7.0 (3H, m), 7.41 (2H, dd), 8.03 (2H, dd)

Example 5

2-(3,4-Dimethoxyphenyl)-6-(4-(4-fluorobenzoyl)-1-piperidinyl)-2-undecylhexanenitrile hydrochloride

TLC Rf = 0.71
MS 592 (M+)
NMR
0.84 (3H, t), 1.2-1.5 (20H, m), 1.6-2.1 (10H, m), 2.9-3.1 (4H, m), 3.4-3.5 (2H, m), 3.6-3.8 (1H, m), 3.77 (3H, s), 3.79 (3H, s), 6.9-7.0 (3H, m), 7.43 (2H, dd), 7.99 (2H, dd)

Example 6

2-(3,4-Dimethoxyphenyl)-6-(4-(4-fluorobenzoyl)-1-piperidinyl)-2-tridecylhexanenitrile hydrochloride

TLC Rf = 0.72
MS 620 (M+)
NMR
0.84 (3H, t), 1.2-1.5 (24H, m), 1.6-2.1 (10H, m), 2.9-3.1 (4H, m), 3.4-3.5 (2H, m), 3.6-3.8 (1H, m), 3.76 (3H, s), 3.79 (3H, s), 6.9-7.0 (3H, m), 7.44 (2H, dd), 8.01 (2H, dd)

Example 7

2-(3,4-Dimethoxyphenyl)-6-(4-(4-fluorobenzoyl)-1-piperidinyl)-2-tetradecylhexanenitrile hydrochloride

TLC Rf = 0.73
MS 634 (M+)
NMR
0.84 (3H, t), 1.2-1.5 (24H, m), 1.6-2.1 (10H, m), 2.9-3.1 (4H, m), 3.4-3.5 (2H, m), 3.6-3.8 (1H, m), 3.76 (3H, s), 3.79 (3H, s) 6.9-7.0 (3H, m), 7.41 (2H, dd), 8.02 (2H, dd)

Example 8

2-Decyl-2-(3,4-dimethoxyphenyl)-6-(4-(α-hydroxy-4-fluorobenzyl)-1-piperidinyl)hexanenitrile hydrochloride

TLC Rf = 0.39
MS 580 (M+)
NMR
0.87 (3H, t), 1.1-1.3 (18H, m), 1.8-3.6 (18H, m), 3.8-3.6 (18H, m), 3.8-3.9 (6H, m), 4.41 (1H, d), 6.8-7.3 (7H, m)

Example 9

2-(3,4-Dimethoxyphenyl)-6-(4-(α-hydroxy-4-fluorobenzyl)-1-piperidinyl)-2-undecylhexanenitrile hydrochloride

TLC Rf = 0.39
MS 594 (M+)
NMR
0.87 (3H, t), 1.1-1.3 (20H, m), 1.8-3.6 (18H, m), 3.8-3.9 (6H, m), 4.41 (1H, d), 6.8-7.3 (7H, m)

Example 10

2-(3,4-Dimethoxyphenyl)-2-dodecyl-6-(4-(α-hydroxy-4-fluorobenzyl)-1-piperidinyl)hexanenitrile hydrochloride

TLC Rf = 0.40
MS 608 (M+)
NMR
0.88 (3H, t), 1.1-1.3 (22H, m), 1.8-3.6 (18H, m), 3.8-3.9 (6H, m), 4.41 (1H, d), 6.8-7.3 (7H, m)

Example 11

2-(3,4-Dimethoxyphenyl)-6-(4-(α-hydroxy-4-fluorobenzyl)-1-piperidinyl)-2-tridecylhexanenitrile hydrochloride

TLC Rf = 0.40
MS 622 (M+)
NMR (fee base)
0.87 (3H, t), 1.1-1.3 (24H, m), 1.8-3.6 (18H, m), 3.8-3.9 (6H, m), 4.44 (1H, d), 6.8-7.3 (7H, m)

Example 12

2-(3,4-Dimethoxyphenyl)-6-(4-(α-hydroxy-4-fluorobenzyl)-1-piperidinyl)-2-tetradecylhexanenitrile hydrochloride

TLC Rf - 0.41
MS 636 (M+)
NMR (free base)
0.87 (3H, t), 1.1-1.3 (26H, m), 1.8-3.6 (18H, m), 3.8-3.9 (6H, m), 4.43 (1H, d), 6.8-7.3 (7H, m)

Example 13

2-Decyl-2-(3,4-dimethoxyphenyl)-7-(4-(4-fluorobenzoyl)-1-piperidinyl)heptanenitrile hydrochloride

TLC Rf = 0.69
MS 592 (M+)
NMR (free base)
0.81 (3H, t), 1.2-1.5 (18H, m), 1.6-2.2 (12H, m), 2.9-3.1 (4H, m), 3.4-3.5 (2H, m), 3.6-3.8 (1H, m), 3.77 (3H, s), 3.78 (3H, s), 6.9-7.0 (3H, m), 7.42 (2H, dd), 8.02 (2H, dd)

Example 14

2-(3,4-Dimethoxyphenyl)-7-(4-(4-fluorobenzoyl)-1-piperidinyl)-2-undecylheptanenitrile hydrochloride

TLC Rf = 0.69
MS 606 (M+)
NMR (free base)
0.84 (3H, t), 1.2-1.5 (20H, m), 1.6-2.2 (12H, m), 2.9-3.1 (4H, m), 3.4-3.5 (2H, m), 3.6-3.8 (1H, m), 3.77 (3H, s), 3.78 (3H, s), 6.9-7.0 (3H, m), 7.41 (2H, dd), 8.04 (2H, dd)

Example 15

2-(3,4-Dimethoxyphenyl)-7-(4-4-fluorobenzoyl)-1-piperidinyl)-2-tridecylheptanenitrile hydrochloride

TLC Rf = 0.74
MS 634 (M+)
NMR (free base)
0.84 (3H, t), 1.2-1.5 (24H, m), 1.6-2.2 (12H, m), 2.9-3.2 (4H, m), 3.4-3.5 (2H, m), 3.6-3.8 (1H, m), 3.77 (3H, s), 3.80 (3H, s), 6.9-7.0 (3H, m), 7.44 (2H, dd), 8.10 (2H, dd)

Example 16

2-(3,4-Dimethoxyphenyl)-7-(4-(4-fluorobenzoyl)-1-piperidinyl)-2-tetradecylheptanenitrile hydrochloride

TLC Rf = 0.72
MS 648 (M+)
NMR
0.85 (3H, t), 1.2-1.5 (26H, m), 1.6-2.2 (12H, m), 2.9-3.1 (4H, m), 3.4-3.5 (2H, m), 3.6-3.8 (1H, m), 3.76 (3H, s), 3.81 (3H, s), 6.9-7.0 (3H, m), 7.44 (2H, dd), 8.10 (2H, dd)

Example 17

2-Decyl-2-(3,4-dimethoxyphenyl)-7-(4-(α-hydroxy-4-fluorobenzyl)-1-piperidinyl)heptanenitrile hydrochloride

TLC Rf - 0.41
MS 594 (M+)
NMR
0.87 (3H, t), 1.1-1.3 (18H, m), 1.8-3.6 (20H, m), 3.8-3.9 (6H, m), 4.39 (1H, d), 6.8-7.3 (7H, m)

Example 18

2-(3,4-Dimethoxyphenyl)-7-(4-(α-hydroxy-4-fluorobenzyl)-1-piperidinyl)-2-undecylheptanenitrile hydrochloride

TLC Rf = 0.39
MS 608 (M+)
NMR
0.89 (3H, t), 1.1-1.3 (20H, m), 1.8-3.6 (20H, m), 3.8-3.9 (6H, m), 4.44 (1H, d), 6.8-7.3 (7H, m)

Example 19

2-(3,4-Dimethoxyphenyl)-2-dodecyl-7-(4-(α-hydroxy-4-fluorobenzyl)-1-piperidinyl)heptanenitrile hydrochloride

TLC Rf = 0.41
MS 622 (M+)
NMR
0.87 (3H, t), 1.1-1.3 (22H, m), 1.8-3.6 (20H, m), 3.8-3.9 (6H, m), 4.39 (1H, d), 6.8-7.3 (7H, m)

Example 20

2-(3,4-Dimethoxyphenyl)-7-(4-(α-hydroxy-4-fluorobenzoyl)-1-piperidinyl)--2-tridecylheptanenitrile hydrochloride

TLC Rf = 0.45
MS 636 (M+)
NMR
0.85 (3H, t), 1.1-1.3 (24H, m), 1.8-3.6 (20H, m), 3.8-3.9 (6H, m), 4.40 (1H, d), 6.8-7.4 (7H, m)

Example 21

2-(3,4-Dimethoxyphenyl)-7-(4-(α-hydroxy-4-fluorobenzoyl)-1-piperidinyl)-2-tetradecylheptanenitrile hydrochloride

TLC Rf - 0.46
MS 650 (M+)
NMR
0.85 (3H, t), 1.1-1.3 (26H, m), 1.8-3.6 (20H, m), 3.8-3.9 (6H, m), 4.41 (3H, d), 6.8-7.3 (7H, m)

Example 22

2-Decyl-2-(3,4-dimethoxyphenyl)-8-(4-(4-fluorobenzoyl)-1-piperidinyl)octanenitrile hydrochloride

TLC Rf = 0.71
MS 606 (M+)
NMR
0.88 (3H, t), 1.2-1.5 (18H, m), 1.6-2.2 (14H, m), 2.9-3.1 (4H, m), 3.4-3.5 (2H, m), 3.6-3.8 (1H, m), 3.78 (3H, s), 3.79 (3H, s), 6.9-7.0 (3H, m), 7.44 (2H, dd), 8.03 (2H, dd)

Example 23

2-(3,4-Dimethoxyphenyl)-7-(4-(4-fluorobenzoyl)-1-piperidinyl)-2-undecylheptanenitrile hydrochloride

TLC Rf = 0.78
MS 620 (M+)
NMR
0.87 (3H, t), 1.2-1.5 (20H, m), 1.6-2.2 (14H, m), 2.9-3.1 (4H, m), 3.4-3.5 (2H, m), 3.6-3.8 (1H, m), 3.78 (3H, s), 3.79 (3H, s), 6.9-7.0 (3H, m), 7.44 (2H, dd), 8.03 (2H, dd)

Example 24

2-(3,4-Dimethoxyphenyl)-8-(4-(4-fluorobenzoyl)-1-piperidinyl)-2-tridecyloctanenitrile

TLC Rf = 0.70
MS 648 (M+)
NMR
0.84 (3H, t), 1.1-1.5 (24H, m), 1.6-2.2 (14H, m), 2.9-3.1 (4H, m), 3.4-3.5 (2H, m) 3.6-3.8 (1H, m), 3.78 (3H, s), 3.79 (3H, s), 6.9-7.0 (3H, m), 7.41 (2H, dd), 8.03 (2H, dd)

Example 25

2-(3,4-Dimethoxyphenyl)-8-(4-(4-fluorobenzoyl)-1-piperidinyl)-2-tetradecyloctanenitrile hydrochloride

TLC Rf = 0.70
MS 662 (M+)
NMR
0.84 (3H, t), 1.1-1.5 (24H, m), 1.6-2.2 (14H, m), 2.9-3.1 (4H, m), 3.4-3.5 (2H, m), 3.6-3.8 (1H, m), 3.78 (3H, s), 3.79 (3H, s), 6.9-7.0 (3H, m), 7.41 (2H, dd), (3H, s), 3.79 (3H, s), 6.9-7.0 (3H, m), 7.41 (2H, dd), 8.03 (2H, dd)

Example 26

2-Decyl-2-(3,4-dimethoxyphenyl)-8-(4-(α-hydroxy-4-fluorobenzyl)-1-piperidinyl)octanenitrile hydrochloride

TLC Rf = 0.41
MS 608 (M+)
NMR
0.85 (3H, t), 1.1-1.3 (18H, m), 1.8-3.6 (22H, m), 3.8-3.9 (6H, m), 4.42 (1H, d), 6.8-7.3 (7H, m)

Example 27

2-(3,4-Dimethoxyphenyl)-8-(4-(α-hydroxy-4-fluorobenzyl)-1-piperidinyl)-2-undecyloctanenitrile hydrochloride

TLC Rf = 0.45
MS 622 (M+)
NMR
0.85 (3H, t), 1.1-1.3 (20H, m), 1.8-3.6 (22H, m), 3.8-3.9 (6H, m), 4.43 (1H, d), 6.8-7.3 (7H, m)

Example 28

2-(3,4-Dimethoxyphenyl)-2-dodecyl-8-(4-(α-hydroxy-4-fluorobenzyl)-1-piperidinyl)octanenitrile hydrochloride

TLC Rf = 0.42
MS 636 (M+)
NMR
0.87 (3H, t), 1.1-1.3 (22H, m), 1.8-3.6 (22H, m), 3.8-3.9 (6H, m), 4.44 (1H, d), 6.8-7.3 (7H, m)

Example 29

2-(3,4-Dimethoxyphenyl)-8-(4-(α-hydroxy-4-fluorobenzyl)-1-piperidinyl)-2-tridecylnitrile hydrochloride

TLC Rf = 0.44
MS 650 (M+)
NMR
0.88 (3H, t), 1.1-1.3 (24H, m), 1.8-3.6 (22H, m), 3.8-3.9 (6H, m), 4.45 (1H, d), 6.8-7.3 (7H, m)

Example 30

2-(3,4-Dimethoxyphenyl)-8-(4-(α-hydroxy-4-fluorobenzyl)-1-piperidinyl)-2-tetradecylnitrile hydrochloride

TLC Rf = 0.42
MS 664 (M+)
NMR
0.88 (3H, t), 1.1-1.3 (26H, m), 1.8-3.6 (22H, m), 3.8-3.9 (6H, m), 4.41 (1H, d), 6.8-7.3 (7H, m)

Example 31

2-(3,4-Dimethoxyphenyl)-2-dodecyl-4-(4-(4-fluorobenzoyl)-1-piperidinyl)butanenitrile hydrochloride

TLC Rf = 0.71
MS 578 (M+)
NMR
0.85 (3H, t), 1.2-1.5 (22H, m), 1.6-2.2 (6H, m), 2.9-3.1 (4H, m), 3.4-3.5 (2H, m), 3.6-3.8 (1H, m), 3.78 (3H, s), 3.79 (3H, s), 6.9-7.0 (3H, m), 7.41 (2H, dd), 8.03 (2H, dd)

Example 32

2-(3,4-Dimethoxyphenyl)-2-dodecyl-4-(4-(4-fluorobenzoyl)-1-piperidinyl)butanenitrile hydrochloride

TLC Rf = 0.37
MS 580 (M+)
NMR
0.87 (3H, t), 1.1-1.3 (22H, m), 1.8-3.6 (12H, m), 3.8-3.9 (6H, m), 4.44 (1H, d), 6.8-7.3 (7H, m)

Example 33

2-Dodecyl-2-(4-methoxyphenyl)-5-(4-(4-fluorobenzoyl)-1-piperidinyl)valeronitrile hydrochloride

TLC Rf = 0.78
MS 562 (M+)
NMR
0.83 (3H, t), 1.2-1.5 (22H, m), 1.6-2.2 (8H, m), 2.9-3.1 (4H, m), 3.4-3.5 (2H, m), 3.6-3.8 (1H, m), 3.79 (3H, s) 7.0-8.0 (8H, m),

Example 34

2-Dodecyl-5-(4-(4-fluorobenzoyl)-1-piperidinyl)-2-phenylvaleronitrile hydrochloride

TLC Rf = 0.79
MS 532 (M+)
NMR
0.83 (3H, t), 1.2-1.5 (22H, m), 1.6-2.2 (8H, m), 2.9-3.1 (4H, m), 3.4-3.5 (2H, m), 3.6-3.8 (1H, m), 7.0-8.0 (9H, m)

Example 35

2-(3,4-Dimethoxyphenyl)-2-dodecyl-5-(4-(α-hydroxybenzyl)-1-piperidinyl)valeronitrile hydrochloride

TLC Rf = 0.40
MS 576 (M+)
NMR
0.88 (3H, t), 1.1-1.3 (22H, m), 1.8-3.6 (16H, m), 3.8-3.9 (6H, m), 4.42 (1H, d), 6.8-7.3 (8H, m)

Example 36

5-(4-(3,4-Dimethoxy-α-hydroxybenzyl)-1-piperidinyl)-2-(3,4-dimethoxyphenyl)-2-dodecylvaleronitrile hydrochloride

TLC Rf = 0.18
MS 636 (M+)
NMR
0.88 (3H, t), 1.1-3.6 (38H, m), 3.8-3.9 (12H, m), 4.3-4.4 (1H, m), 6.8-7.3 (6H, m)

Example 37

2-(3,4-Dimethoxyphenyl)-2-dodecyl-5-(4-phenyl-4-hydroxy-1-piperidinyl)valeronitrile hydrochloride

TLC Rf = 0.45
MS 562 (M+)
NMR (free base)
0.88 (3H, t), 1.0-2.9 (36H, m), 3.87 (3H, s), 3.93 (3H, s), 6.8-7.0 (3H, m), 7.2-7.6 (5H, m)

Example 38

2-(3,4-Dimethoxyphenyl)-2-dodecyl-5-(4-phenyl-1-piperazinyl)valeronitrile hydrochloride

TLC Rf = 0.51
MS 547 (M+)
NMR (free base)
0.88 (3H, t), 1.0-2.1 (26H, m), 2.35 (2H, t), 2.50(4H, t), 3.18 (4H, t), 3.87 (3H, s), 3.90 (3H, s), 6.8-7.3 (8H, m)

Example 39

5-(4-Benzhydryl-1-piperazinyl)-2-(3,4-dimethoxyphenyl)-2-dodecylvaleronitrile hydrochloride

TLC Rf = 0.47
MS 637 (M+)
NMR (free base)
0.88 (3H, t), 1.0-2.5 (36H, m), 3.84 (3H, s), 3.86 (3H, s), 4.20 (1H, s), 6.8-7.4 (13H, m)

Example 40

2-(3,4-Dimethoxyphenyl)-2-dodecyl-5-(4-(4-fluorophenyl)-methylene-1-piperidinyl)valeronitrile hydrochloride

TLC Rf = 0.75
MS 570 (M+)

NMR
0.85 (3H, t), 1.1-3.8 (36H, m), 3.9-4.0 (6H, m), 6.4-6.5 (1H, m), 6.8-7.4 (7H, m)

Example 41

5-(4-(4-Fluorobenzoyl)-1-piperidinyl)-2-dodecyl-2-(3-trifluoromethylphenyl)valeronitrile hydrochloride

TLC Rf - 0.66
MS 600 (M+)
NMR
0.91 (3H, t, 1.1-1.5 (22H, m), 1.6-2.2 (8H, m), 2.9-3.1 (4H, m), 3.4-3.5 (2H, m) 3.6-3.8 (1H, m), 7.18 (2H, dd), 7.6-7.7 (3H, m), 7.94 (2H, dd)

Example 42

5-(4-(5H-Dibenzo[a,d]cyclohepten-5-ylidene)-1-piperidinyl)-2-dodecyl-2-(3,4-dimethoxyphenyl)valeronitrile hydrochloride

TLC Rf = 0.88
MS 658 (M+)
NMR (free base)
0.87 (3H, t), 1.1-2.5 (35H, m), 3.86 (3H, s), 3.88 (3H, s), 6.8-7.0 (3H, m), 6.90 (1H, m), 7.1-7.4 (8H, m)

Example 43

2-(3,4-Dimethoxyphenyl)-5-(4-(4-fluorobenzyl)-1-piperazinyl)-2-dodecylvaleronitrile hydrochloride

TLC Rf = 0.34
MS 579 (M+)
NMR
0.88 (3H, t), 1.1-3.4 (38H, m), 6.8-8.0 (7H, m)

Example 44

5-(4-Benzyl-1-piperazinyl)-2-(3,4-dimethoxyphenyl-2-dodecylvaleronitrile hydrochloride

TLC Rf = 0.39
MS 561 (M+)
NMR
0.84 (3H, t), 1.1-3.4 (38H, m), 6.8-7.4 (8H, m)

Example 45

4-(3,4-Dimethoxyphenyl)-1-(4-(4-fluorobenzoyl)-1-piperidinyl)-hexadecanenitrile hydrochloride

TLC Rf = 0.79
MS 567 (M+)
NMR (free base)
0.87 (3H, t), 1.1-3.9 (38H, m), 6.8-7.4 (8H, m)

Example 46

Methyl 2-(3,4-Dimethoxyphenyl)-2-dodecyl-2-(3-(4-(4-fluorobenzoyl)-1-piperidinyl)-1-propyl)acetate hydrochloride

TLC Rf = 0.61
MS 625 (M+)
NMR (free base)
0.84 (3H, t), 1.2-1.5 (22H, m), 1.8-2.1 (8H, m), 2.9-3.1 (4H, m), 3.4-3.5 (2H, m), 3.6-3.8 (1H, m), 3.61 (3H, s), 3.76 (3H, s), 3.79 (3H, s), 6.9-7.0 (3H, m), 7.40 (2H, dd), 8.07 (2H, dd)

Example 47

2-(3,4-Dimethoxyphenyl)-2-dodecyl-2(3-(4-(4-fluorobenzoyl)-1-piperidinyl)-1-propyl)acetic acid hydrochloride

TLC Rf = 0.24
MS 612 (MH+)
NMR (DMSO-d6)
0.84 (3H, t), 1.2-1.5 (22H, m), 1.8-2.1 (8H, m), 2.9-3.1 (4H, m), 3.4-3.5 (2H, m), 3.6-3.8 (1H, m), 3.75 (3H, s), 3.78 (3H, s), 6.9-7.0 (3H, m), 7.32 (2H, dd), 8.12 (2H, dd)

Example 48

2-(3,4-Dimethoxyphenyl)-2-dodecyl-2-(3-(4-(4-fluorobenzoyl)-1-piperidinyl)-1-propyl)aacetamide hydrochloride

TLC Rf = 0.44
MS 610 (M+)
NMR
0.87 (3H, t), 1.2-1.5 (22H, m), 1.8-2.1 (8H, m), 2.9-3.1 (4H, m), 3.4-3.5 (2H, m), 3.6-3.8 (1H, m), 3.82 (3H, s), 3.88 (3H, s), 6.9-7.0 (3H, m), 7.41 (2H, dd), 8.01 (2H, dd)

Example 49

2-(3,4-Dimethoxyphenyl)-7-(4-(α-hydroxy-4-fluorobenzoyl)-1-piperidinyl)-2-tridecylheptanenitrile hydrochloride

TLC Rf = 0.67
MS 580 (M+)
NMR
0.82 (3H, t), 1.1-3.4 (34H, m), 3.58 (3H, s), 3.86 (3H, s), 3.88 (3H, s), 6.9-8.0 (7H, m)

Example 50

N-(3-(4-(4-Fluorobenzoyl)-1-propyl)-N-methyl 4-cyano-4-(3,4-dimethoxyphenyl)hexadecylamine hydrochloride

TLC Rf = 0.32
MS 582 (M+)
NMR
0.87 (3H, t), 1.1-3.4 (34H, m), 3.58 (3H, s), 3.86 (3H, s), 3.88 (3H, s), 4.25 (1H, d), 6.9-8.0 (7H, m)

Test Example 1

As test animals, four male SHR (spontaneous hypertensive rats, weighing 400 to 440 g) that were sufficiently adapted for feeding and in which hypertension was confirmed were used.

Physiological saline aqueous solution (ml/kg) containing 2.5% Nicolle of sample and 2.5% ethanol was intravenously administered at once. Blood pressure after the administration was measured by tail-cuff method.

The results are shown below

| Example | Dose (mg/kg) | Decrease in Blood Pressure (mmHg) | |
|---|---|---|---|
| | | 30 minutes | 4 hours |
| 3 | 10 | - 62 | - 62 |
| 4 | 10 | - 135 | - 80 |
| 5 | 10 | - 125 | - 53 |
| 6 | 10 | - 61 | - 31 |
| 7 | 10 | - 34 | - 34 |
| 10 | 10 | - 78 | - 79 |
| 19 | 10 | - 62 | - 74 |
| 28 | 10 | - 41 | - 45 |
| 37 | 3 | - 67 | - 33 |
| 39 | 10 | - 45 | - 42 |
| 40 | 10 | - 35 | - 22 |
| 42 | 10 | - 40 | - 70 |
| 44 | 3 | - 23 | + 2 |
| 49 | 10 | - 125 | - 93 |
| 50 | 10 | - 125 | - 101 |

Example 50A(illustrative)

4-(5H-Dibenzo[a,d]cyclohepten-5-ylidene)-1-hexylpiperidinne hydrochloride

This Example illustrates the experimental procedure used in the preparation of the compounds of Examples 51 to 152 which follow:

A solution of 273 mg (1 mmol) of 4-(5H-dibenzo[a,d]cyclohepten-5-ylidene)-1-hexylpiperidine, 165 mg (1 mmol) of 1-bromohexane, 745 mg (5 mmols) of sodium iodide and 414 mg (3 mmols) of potassium carbonate in 20 ml of methyl isobutyl ketone was stirred and refluxed at 120°C overnight on an oil bath. After the reaction, the mixture was washed by adding 20 ml of water thereto. Then the organic phase was separated and the solvlent was distilled off under reduced pressure. After purifying the silica gel column chromatography (eluent: methanol/chloroform, 1/100 - 1/50), the product was converted into the hydrochloride with an equimolar hydrogen chloride/dioxane solution.

Amount yielded 180 mg

Yield 46%

TLC Rf = 0.68

MS 357 (M+)

NMR

0.83 (3H,t), 1.2-1.4 (6H, m), 1.7-1.9 (2H, m), 2.31 (2H, dd), 2.53 (2H, d), 2.7-2.8 (2H, m), 3.14 (2H, dd), 3.38 (2H, dd), 3.38 (2H, d), 6.92 (2H, s), 7.2-7.4 (8H, m)

Example 51

3-(4-(5H-Dibenzo[a,d]cyclohepten-5-ylidene)-1-piperidinyl)-1-phenylpropane hydrochloride:

Amount yielded 330 mg

Yield 77%
TLC Rf = 0.50
MS 391 (M+)
NMR
2.1-2.4 (4H, m), 2.51 (2H, d), 2.65 (2H, t), 2.7-2.9 (2H, m), 3.12 (2H, dd), 3.38 (2H, d), 6.90 (2H, s), 7.1-7.4 (13H, m)

Example 52

4-(4-(5H-Dibenzo[a,d]cyclohepten-5-ylidene)-1-piperidinyl)-1-phenylbutane hydrochloride:

Amount yielded 180 mg
Yield 41%
TLC Rf = 0.50
MS 405 (M+)
NMR
1.4-1.9 (4H, m), 2.28 (2H, dd), 2.52 (2H, d), 2.61 (2H, t), 2.7-2.8 (2H, m), 3.12 (2H, dd), 3.35 (2H, d), 6.90 (2H, s), 7.1-7.4 (13H, m)

Example 53

5-(4-(5H-Dibenzo[a,d]cyclohepten-5-ylidene)-1-piperidinyl)-1-phenylpentane hydrochloride:

Amount yielded 110 mg
Yield 24%
TLC Rf = 0.55
MS 419 (M+)
NMR
1.2-1.9 (6H, m), 2.25 (2H, dd), 2.52 (2H, d), 2.60 (2H, t), 2.7-2.8 (2H, m), 3.08 (2H, dd), 3.35 (2H, d), 6.90 (2H, s), 7.1-7.4 (13H, m)

Example 54

6-(4-(5H-Dibenzo[a,d]cyclohepten-5-ylidene)-1-piperidinyl)-1-phenylhexane hydrochloride:

Amount yielded 315 mg
Yield 67%
TLC Rf = 0.56
MS 433 (M+)
NMR
1.1-1.9 (8H, m), 2.26 (2H, dd), 2.56 (2H, d), 2.61 (2H, t), 2.7-2.8 (2H, m), 3.10 (2H, dd), 3.35 (2H, d), 6.91 (2H, s), 7.1-7.4 (13H, m)

Example 55

7-(4-(5H-Dibenzo[a,d]cyclohepten-5-ylidene)-1-piperidinyl)-1-phenylheptane hydrochloride:

Amount yielded 267 mg
Yield 55%
TLC Rf = 0.56
MS 447 (M+)
NMR
1.1-1.9 (10H, m), 2.25 (2H, dd), 2.55 (2H, d), 2.65 (2H, t), 2.7-2.8 (2H, m), 3.07 (2H, dd), 3.32 (2H, d), 6.90 (2H, s), 7.1-7.4 (13H, m)

Example 56

3-(4-(5H-Dibenzo[a,d]cyclohepten-5-ylidene)-1-piperidinyl)-1-phenoxypropane hydrochloride:

Amount yielded 2.15 g
Yield 48%
TLC Rf = 0.58
MS 407 (M+)
NMR (free base)
1.97 (2H, tt), 2.1-2.5 (6H, m), 2.54 (2H, t), 2.6-2.7 (2H, m), 3.97 (2H, dd), 6.86 (2H, d), 6.90 (2H, s), 7.1-7.4 (11H, m)

Example 57

4-(4-(5H-Dibenzo[a,d]cyclohepten-5-ylidene)-1-piperidinyl)-1-phenoxybutane hydrochloride:

Amount yielded 1.18 g
Yield 86%
TLC Rf = 0.61
MS 421 (M+)
NMR (free base)
1.8-2.7 (14H, m), 3.96 (2H, t), 6.87 (2H, d), 6.90 (2H, s), 7.1-7.4 (11H, m)

Example 58

2-(4-(5H-dibenzo[a,d]cyclohepten-5-ylidene)-1-piperidinyl)-1-phenylthioethane hydrochloride:

Amount yielded 0.97 g
Yield 87%
TLC Rf = 0.55
MS 409 (M+)
NMR (free base)
2.0-2.6 (10H, m), 2.78 (2H, t), 6.86 (2H, s), 7.1-7.4 (11H, m)

Example 59

3-(4-(5H-Dibenzo[a,d]cyclohepten-5-ylidene)-1-piperidinyl)-1-phenylthiopropane hydrochloride:

Amount yielded 0.85 g
Yield 74%
TLC Rf = 0.62
MS 423 (M+)
NMR (free base)
1.73 (2H, tt), 2.0-2.6 (10H, m), 2.80 (2H, t), 6.88 (2H, d), 7.1-7.4 (11H, m)

Example 60

4-(4-(5H-Dibenzo[a,d]cyclohepten-5-ylidene)-1-piperidinyl)-1-phenylthiobutane hydrochloride:

Amount yielded 0.85 g
Yield 72%
TLC Rf = 0.62
MS 437 (M+)

NMR (free base)
1.6-2.6 (14H, m), 2.80 (2H, t), 6.88 (2H, d), 7.1-7.4 (11H, m)

Example 61

4-(5H-Dibenzo[a,d]cyclohepten-5-ylidene)-1-(2-(2-nitrobenzenesulfonylamino)ethyl)piperidine hydrochloride:

TLC Rf = 0.72
MS 502 (M+)

Example 62

1-(2-(2-Aminobenzenesulfonyl)amino)ethyl)-4-(5H-dibenzo[a,d]-cyclohepten-5-ylidene)piperidine hydrochloride:

TLC Rf = 0.51
MS 472 (M+)

Example 63

4-(5H-Dibenzo[a,d]cyclohepten-5-ylidene)-1-(2-(2-ethoxycarbonylbenzenesulfonylamino)ethyl)piperidine hydrochloride:

TLC Rf = 0.68
MS 544 (M+)

Example 64

3-(2-( 4-(5H-Dibenzo[a,d]cyclohepten-5-ylidene)-1-piperidinyl)ethyl)-2,4(1H,3H)quinazolinedione hydrochloride:

TLC Rf = 0.85
MS 462 (M+)

Example 65

5,6-Benzo-2,4-diaza(2-(4-(5H-dibenzo[a,d]cyclohepten-5-ylidene)-1-piperidinyl)ethyl)tetrahydrothiopyrane 1,1-dioxide hydrochloride

TLC Rf = 0.91
MS 498 (M+)

Example 66

5-(4-(5H-Dibenzo[a,d]cyclohepten-5-ylidene)-1-piperidinyl)-2-(3,4-dimethoxyphenyl)-2-isopropylvaleronitrile hydrochloride:

TLC Rf = 0.92
MS 532 (M+)
NMR
0.77 (3H, d), 1.18 (3H, d), 1.6-3.3 (15H, m), 3.86 (3H, s), 3.92 (3H, s), 6.8-7.4 (11H, m)

Example 67

3-(4-(5H-Dibenzo[a,d]cyclohepten-5-ylidene)-1-piperidinyl)propyl-4-fluorophenylsulfoxide hydrochloride:

TLC Rf = 0.78
MS 457 (M+)

Example 68

3-(4-(5H-Dibenzo[a,d]cyclohepten-5-ylidene)-1-piperidinyl)propyl-4-fluorophenylsulfone hydrochloride:

TLC Rf = 0.62
MS 473 (M+)

Example 69

4-(5H-Dibenzo[a,d]cyclohepten-5-ylidene)-1-(3-(2-aminophenylthio)propyl)piperidine hydrochloride:

TLC Rf = 0.84
MS 439 (M+)

Example 70

4-(5H-Dibenzo[a,d]cyclohepten-5-ylidene)-1-(2-( benzoylamino)ethyl)piperidine hydrochloride:

TLC Rf = 0.84
MS 420 (M+)

Example 71

4-(5H-Dibenzo[a,d]cyclohepten-5-ylidene)-1-(2-( phenylcarbamoylamino)ethyl)piperidine hydrochloride:

TLC Rf = 0.55
MS 435 (M+)

Example 72

1-(3-(2-Cinnamoylaminophenylthio)- propyl)-4-(5H-dibenzo[a,d]cyclohepten-5-ylidene)piperidine hydrochloride:

TLC Rf = 0.66
MS 568 (M+)
NMR (free base)
1.74 (2H, tt), 2.0-2.6 (8H, m), 2.80 (2H, t), 6.59 (1H, d), 6.88 (2H, s), 7.0-7.6 (16H, m), 7.75 (1H, d), 8.5 (1H, d), 8.68 (1H, bs)

Example 73

5-(4-(5H-Dibenzo[a,d]cyclohepten-5-ylidene)-1-piperidinyl)-2-(3,4,5-trimethoxyphenyl)-2-isopropylvaleronitrile hydrochloride:

TLC Rf = 0.80
MS 563 (M+)

Example 74

2-(3-(4-(5H-Dibenzo[a,d]cyclohepten-5-ylidene)-1-piperidinyl)propyl)-2-phenyl-1,3-dithiane-1,1,3,3-tetroxide hydrochloride:

TLC Rf = 0.48
MS 573 (M+)

Example 75

2-(3,4-Dimethoxyphenyl)-2-(3-(4-(5H-Dibenzo[a,d]cyclohepten-5-ylidene)-1-piperidinyl)- propyl)-1,3-dithiane-1,1,3,3-tetroxide hydrochloride:

TLC Rf = 0.48
MS 573 (M+)

Example 76

5-(4-(5H-Dibenzo[a,d]cyclohepten-5-ylidene)-1-piperidinyl)-2-(3,4-dichlorophenyl)-2-isopropylvaleronitrile hydrochloride:

TLC Rf = 0.94
MS 540 (M+)

Example 77

2-(3-Benzoylphenyl)-5-(4-(5H-Dibenzo[a,d]cyclohepten-5-ylidene)-1-piperidinyl)-2-methylvaleronitrile hydrochloride:

TLC Rf = 0.88
MS 548 (M+)

Example 78

5-(4-(5H-Dibenzo[a,d]cyclohepten-5-ylidene)-1-piperidinyl)-2,2-diphenylvaleronitrile hydrochloride:

TLC Rf = 0.74
MS 506 (M+)

Example 79

4-(4-(5H-Dibenzo[a,d]cyclohepten-5-ylidene)-1-piperidinyl)-3′,4′-dimethoxybutyrophenone hydrochloride:

TLC Rf = 0.61
MS 479 (M+)

Example 80

6-(4-(5H-Dibenzo[a,d]cyclohepten-5-ylidene)-1-piperidinyl)-2-phenylhexanenitrile hydrochloride:

TLC Rf = 0.86
MS 430 (M+)

Example 81

6-(4-(5H-Dibenzo[a,d]cyclohepten-5-ylidene)-1-piperidinyl)-2-isopropyl-2-phenylhexanenitrile hydrochloride:

TLC Rf = 0.88
MS 472 (M+)

Example 82

6-(4-(5H-Dibenzo[a,d]cyclohepten-5-ylidene)-1-piperidinyl)-2-(3,4-dimethoxyphenyl)-2-isopropylhexanenitrile hydrochloride:

TLC Rf = 0.81
MS 546 (M+)

Example 83

7-(4-(5H-Dibenzo[a,d]cyclohepten-5-ylidene)-1-piperidinyl)-2-phenylheptanenitrile hydrochloride:

TLC Rf = 0.84
MS 444 (M+)

Example 84

7-(4-(5H-Dibenzo[a,d]cyclohepten-5-ylidene)-1-piperidinyl)-2-isopropyl-2-phenylheptanenitrile hydrochloride:

TLC Rf = 0.84
MS 486 (M+)

Example 85

7-(4-(5H-Dibenzo[a,d]cyclohepten-5-ylidene)-1-piperidinyl)-2-(3,4-dimethoxyphenyl)-2-isopropylheptanenitrile hydrochloride:

TLC Rf = 0.86
MS 560 (M+)

Example 86

2-(3-Chloropropyl)-5-(4-(5H-dibenzo[a,d]cyclohepten-5-ylidene)-1-piperidinyl)-2-phenylvaleronitrile hydrochloride:

TLC Rf = 0.92
MS 506 (M+)

Example 87

5-(4-(5H-Dibenzo[a,d]cyclohepten-5-ylidene)-1-piperidinyl)-2-phenyl-2-phenylthiovaleronitrile hydrochloride:

TLC Rf = 0.81
MS 538 (M+)

Example 88

5-(4-(5H-Dibenzo[a,d]cyclohepten-5-ylidene)-1-piperidinyl)-2-(3,4-dimethoxyphenyl)-2-phenylthiovaleronitrile hydrochloride:

TLC Rf = 0.91
MS 598 (M+)

Example 89

5-(4-(5H-Dibenzo[a,d]cyclohepten-5-ylidene)-1-piperidinyl)-2-(1-naphthyl)valeronitrile hydrochloride:

TLC Rf = 0.85
MS 580 (M+)

Example 90

5-(4-(5H-Dibenzo[a,d]cyclohepten-5-ylidene)-1-piperidinyl)-2-(1-naphthyl)-2-isopropylvaleronitrile hydrochloride:

TLC Rf = 0.90
MS 522 (M+)

Example 91

5-(4-(5H-Dibenzo[a,d]cyclohepten-5-ylidene)-1-piperidinyl)-2-(2-naphthyl)valeronitrile hydrochloride:

TLC Rf = 0.85
MS 480 (M+)

Example 92

5-(4-(5H-Dibenzo[a,d]cyclohepten-5-ylidene)-1-piperidinyl)-2-(2-naphthyl)-2-isopropylvaleronitrile hydrochloride:

TLC Rf = 0.87
MS 522 (M+)

Example 93

5-(4-(5H-Dibenzo[a,d]cyclohepten-5-ylidene)-1-piperidinyl)-2-(3-trifluoromethylphenyl)valeronitrile hydrochloride:

TLC Rf = 0.72
MS 498 (M+)

Example 94

5-(4-(5H-Dibenzo[a,d]cyclohepten-5-ylidene)-1-piperidinyl)-2-isopropyl-2-(3-trifluoromethylphenyl)valeronitrile hydrochloride:

TLC Rf = 0.75
MS 540 (M+)

Example 95

8-(4-(5H-Dibenzo[a,d]cyclohepten-5-ylidene)-1-piperidinyl)-2-phenyloctanenitrile hydrochloride:

TLC Rf = 0.84
MS 472 (M+)

Example 96

8-(4-(5H-Dibenzo[a,d]cyclohepten-5-ylidene)-1-piperidinyl)-2-isopropyl-2-phenyloctanenitrile hydrochloride:

TLC Rf = 0.88
MS 514 (M+)

Example 97

8-(4-(5H-Dibenzo[a,d]cyclohepten-5-ylidene)-1-piperidinyl)-2-(3,4-dimethoxyphenyl)-2-isopropyloctanenitrile hydrochloride:

TLC Rf = 0.82
MS 574 (M+)

Example 98

1-(3-(4-(5H-Dibenzo[a,d]cyclohepten-5-ylidene)-1-piperidinyl)propyl)-1-indanecarbonitrile hydrochloride:

TLC Rf = 0.90
MS 456 (M+)

Example 99

1-(3-(4-(5H-Dibenzo[a,d]cyclohepten-5-ylidene)-1-piperidinyl)propyl)-5,6-dimethoxy-1-indanecarbonitrile hydrochloride:

TLC Rf = 0.85
MS 516 (M+)

Example 100

5-(4-(5H-Dibenzo[a,d]cyclohepten-5-ylidene)-1-piperidinyl)-2-(1-methylpyrrol-2-yl)valeronitrile hydrochloride:

TLC Rf = 0.61
MS 433 (M+)

Example 101

5-(4-(5H-Dibenzo[a,d]cyclohepten-5-ylidene)-1-piperidinyl)-2-isopropyl-2-(1-methylpyrrol-2-yl)valeronitrile hydrochloride:

TLC Rf = 0.71
MS 475 (M+)

Example 102

5-(4-(5H-Dibenzo[a,d]cyclohepten-5-ylidene)-1-piperidinyl)-2-isopropyl-2-(pyrrol-2-yl)valeronitrile hydrochloride:

TLC Rf = 0.55
MS 461 (M+)

Example 103

5-(4-(5H-Dibenzo[a,d]cyclohepten-5-ylidene)-1-piperidinyl)-2-(3-(α-hydroxybenzyl)phenyl)-2-methylvaleronitrile hydrochloride:

TLC Rf = 0.51
MS 550 (M+)

Example 104

2-(3-Benzoylphenyl)-6-(4-(5H-Dibenzo[a,d]cyclohepten-5-ylidene)-1-piperidinyl)-2-methylhexanenitrile hydrochloride:

TLC Rf = 0.88
MS 562 (M+)

Example 105

6-(4-(5H-Dibenzo[a,d]cyclohepten-5-ylidene)-1-piperidinyl)-2-(3-(α-hydroxybenzyl)phenyl)-2-methylhexanenitrile hydrochloride:

TLC Rf = 0.52
MS 564 (M+)

Example 106

2-(3-Benzoylphenyl)-7-(4-(5H-Dibenzo[a,d]cyclohepten-5-ylidene)-1-piperidinyl)-2-methylheptanenitrile hydrochloride:

TLC Rf = 0.91
MS 576 (M+)

Example 107

7-(4-(5H-Dibenzo[a,d]cyclohepten-5-ylidene)-1-piperidinyl)-2-(3-(α-hydroxybenzyl)phenyl)-2-methylheptanenitrile hydrochloride:

TLC Rf = 0.52

MS 578 (M+)

Example 108

2-(3-Benzoylphenyl)-8-(4-(5H-Dibenzo[a,d]cyclohepten-5-ylidene)-1-piperidinyl)-2-methyloctanenitrile hydrochloride:

TLC Rf = 0.90
MS 590 (M+)

Example 109

8-(4-(5H-Dibenzo[a,d]cyclohepten-5-ylidene)-1-piperidinyl)-2-(3-(α-hydroxybenzyl)phenyl)-2-methyloctanenitrile hydrochloride:

TLC Rf = 0.61
MS 592 (M+)

Example 110

5-(4-(5H-Dibenzo[a,d]cyclohepten-5-ylidene)-1-piperidinyl)-2-methyl-2-phenylvaleronitrile hydrochloride:

TLC Rf = 0.91
MS 544 (M+)

Example 111

5-(4-(5H-Dibenzo[a,d]cyclohepten-5-ylidene)-1-piperidinyl)-2-(3,4-dimethoxyphenyl)-2-methylvaleronitrile hydrochloride:

TLC Rf = 0.85
MS 504 (M+)

Example 112

5-(4-(5H-Dibenzo[a,d]cyclohepten-5-ylidene)-1-piperidinyl)-2-ethyl-2-phenylvaleronitrile hydrochloride:

TLC Rf = 0.92
MS 458 (M+)

Example 113

5-(4-(5H-Dibenzo[a,d]cyclohepten-5-ylidene)-1-piperidinyl)-2-(3,4-dimethoxyphenyl)-2-ethylvaleronitrile hydrochloride:

TLC Rf = 0.90
MS 518 (M+)

Example 114

5-(4-(5H-Dibenzo[a,d]cyclohepten-5-ylidene)-1-piperidinyl)-2-propyl-2-phenylvaleronitrile hydrochloride:

TLC Rf = 0.93
MS 472 (M+)

Example 115

5-(4-(5H-Dibenzo[a,d]cyclohepten-5-ylidene)-1-piperidinyl)-2-(3,4-dimethoxyphenyl)-2-propylvaleronitrile hydrochloride:

TLC Rf = 0.91
MS 532 (M+)

Example 116

2-Butyl-5-(4-(5H-Dibenzo[a,d]cyclohepten-5-ylidene)-1-piperidinyl)-2-phenylvaleronitrile hydrochloride:

TLC Rf = 0.95
MS 486 (M+)

Example 117

2-Butyl-5-(4-(5H-Dibenzo[a,d]cyclohepten-5-ylidene)-1-piperidinyl)-1-piperidinyl)-2-(3,4-dimethoxyphenyl)-valeronitrile hydrochloride:

TLC Rf = 0.90
MS 546 (M+)

Example 118

5-(4-(5H-Dibenzo[a,d]cyclohepten-5-ylidene)-1-piperidinyl)-2-pentyl-2-phenylvaleronitrile hydrochloride:

TLC Rf = 0.95
MS 500 (M+)

Example 119

5-(4-(5H-Dibenzo[a,d]cyclohepten-5-ylidene)-1-piperidinyl)-2-(3,4-dimethoxyphenyl)-2-phenylvaleronitrile hydrochloride:

TLC Rf = 0.92
MS 560 (M+)

Example 120

5-(4-(5H-Dibenzo[a,d]cyclohepten-5-ylidene)-1-piperidinyl)-2-hexyl-2-phenylvaleronitrile hydrochloride:

TLC Rf = 0.95
MS 514 (M+)

Example

5-(4-(5H-Dibenzo[a,d]cyclohepten-5-ylidene)-1-piperidinyl)-2-(3,4-dimethoxyphenyl)-2-hexylvaleronitrile hydrochloride:

TLC Rf = 0.92
MS 574 (M+)

Example 122

5-(4-(5H-Dibenzo[a,d]cyclohepten-5-ylidene)-1-piperidinyl)-2-heptyl-2-phenylvaleronitrile hydrochloride:

TLC Rf = 0.95
MS 528 (M+)

Example 123

5-(4-(5H-Dibenzo[a,d]cyclohepten-5-ylidene)-1-piperidinyl)-2-(3,4-dimethoxyphenyl)-2-heptylvaleronitrile hydrochloride:

TLC Rf = 0.91
MS 588 (M+)

Example 124

5-(4-(5H-Dibenzo[a,d]cyclohepten-5-ylidene)-1-piperidinyl)-2-octyl-2-phenylvaleronitrile hydrochloride:

TLC Rf = 0.94
MS 542 (M+)

Example 125

5-(4-(5H-Dibenzo[a,d]cyclohepten-5-ylidene)-1-piperidinyl)-2-(3,4-dimethoxyphenyl)-2-octylvaleronitrile hydrochloride:

TLC Rf = 0.94
MS 602 (M+)

Example 126

5-(4-(5H-Dibenzo[a,d]cyclohepten-5-ylidene)-1-piperidinyl)-2-nonyl-2-phenylvaleronitrile hydrochloride:

TLC Rf = 0.95
MS 556 (M+)

Example 127

5-(4-(5H-Dibenzo[a,d]cyclohepten-5-ylidene)-1-piperidinyl)-2-(3,4-dimethoxyphenyl)-2-nonylvaleronitrile hydrochloride:

TLC Rf = 0.93
MS 616 (M+)

Example 128

2-Decyl-5-(4-(5H-Dibenzo[a,d]cyclohepten-5-ylidene)-1-piperidinyl)-2-phenylvaleronitrile hydrochloride:

TLC Rf = 0.95
MS 570 (M+)

Example 129

2-Decyl-5-(4-(5H-Dibenzo[a,d]cyclohepten-5-ylidene)-1-piperidinyl)-2-(3,4-dimethoxyphenyl)valeronitrile hydrochloride:

TLC Rf = 0.94
MS 630 (M+)

Example 130

3-(4-(5H-Dibenzo[a,d]cyclohepten-5-ylidene)-1-piperidinyl)-1-hydroxy-1-phenylpropane hydrochloride:

TLC Rf = 0.35
MS 407 (M+)

Example 131

4-(4-(5H-Dibenzo[a,d]cyclohepten-5-ylidene)-1-piperidinyl)butyrophenone hydrochloride:

TLC Rf = 0.75
MS 419 (M+)

Example 132

4-(4-(5H-Dibenzo[a,d]cyclohepten-5-ylidene)-1-piperidinyl)-1-hydroxy-1-phenylbutane hydrochloride:

TLC Rf = 0.39
MS 421 (M+)

Example 133

5-(4-(5H-Dibenzo[a,d]cyclohepten-5-ylidene)-1-piperidinyl)valerophenone hydrochloride:

TLC Rf = 0.76
MS 433 (M+)

Example 134

5-(5H-Dibenzo[a,d]cyclohepten-5-ylidene)-1-piperidinyl)-1-hydroxy-1-phenylpentane hydrochloride:

TLC Rf = 0.78
MS 447 (M+)

Example 135

3-(4-(5H-Dibenzo[a,d]cyclohepten-5-ylidene)-1-piperidinyl)-1-hydroxy-1-(4-fluorophenyl)propane hydrochloride:

TLC Rf = 0.44
MS 425 (M+)

Example 136

4-(4-(5H-Dibenzo[a,d]cyclohepten-5-ylidene)-1-piperidinyl)-1-hydroxy-1-(4-fluorophenyl)butane hydrochloride:

TLC Rf = 0.45
MS 439 (M+)

Example 137

5-(4-(5H-Dibenzo[a,d]cyclohepten-5-ylidene)-1-piperidinyl)-4′-fluorovalerophenone hydrochloride:

TLC Rf = 0.84
MS 451 (M+)

Example 138

5-(4-(5H-Dibenzo[a,d]cyclohepten-5-ylidene)-1-piperidinyl)-1-hydroxy-1-(4-fluorophenyl)pentane hydrochloride:

TLC Rf = 0.51
MS 453 (M+)

Example 139

5-(4-(5H-Dibenzo[a,d]cyclohepten-5-ylidene)-1-piperidinyl)-2-phenylvaleronitrile hydrochloride:

TLC Rf = 0.86
MS 440 (M+)

Example 140

5-(4-(5H-Dibenzo[a,d]cyclohepten-5-ylidene)-1-piperidinyl)-2-isopropyl-2-phenylvaleronitrile hydrochloride:

TLC Rf = 0.82
MS 488 (M+)

Example 141

5-(4-(5H-Dibenzo[a,d]cyclohepten-5-ylidene)-1-piperidinyl)-2-(3,4-dimethoxyphenyl)valeronitrile hydrochloride:

TLC Rf = 0.75
MS 500 (M+)

Example 142

2-(3-(4-(5H-Dibenzo[a,d]cyclohepten-5-ylidene)-1-piperidinyl)-propyl-2-(4-fluorophenyl)-1,3-dioxolane hydrochloride:

TLC Rf = 0.68
MS 481 (M+)

Example 143

4-(4-(5H-Dibenzo[a,d]cyclohepten-5-ylidene)-1-piperidinyl)-4′-fluorobutyrophenone hydrochloride:

TLC Rf = 0.82
MS 437 (M+)

Example 144

5-(4-(5H-Dibenzo[a,d]cyclohepten-5-ylidene)-1-piperidinyl)-2-isopropyl-2-(3,4,5-trimethoxyphenyl)valeronitrile hydrochloride:

TLC Rf = 0.66
MS 562 (M+)

Example 145

5-(4-(5H-Dibenzo[a,d]cyclohepten-5-ylidene)-1-piperidinyl)-2-(3-fluorophenyl)-2-isopropylvaleronitrile hydrochloride

TLC Rf= 0.71
MS 500 (M+)

Example 146

5-(4-(5H-Dibenzo[a,d]cyclohepten-5-ylidene)-1-piperidinyl)-2-isopropyl-2-(3-methoxyphenyl)valeronitrile hydrochloride

TLC Rf = 0.64
MS 512 (M+)

Example 147

5-(4-(5H-Dibenzo[a,d]cyclohepten-5-ylidene)-1-piperidinyl)-2-isopropyl-2-(3-methyl phenyl)valeronitrile hydrochloride

TLC Rf = 0.68
MS 496 (M+)

Example 148

5-(4-(5H-Dibenzo[a,d]cyclohepten-5-ylidene)-1-piperidinyl)-2-isopropyl-2-(2-trifluoromethylphenyl)valeronitrile hydrochloride

TLC Rf = 0.78
MS 549 (M+)

Example 149

5-(4-(5H-Dibenzo[a,d]cyclohepten-5-ylidene)-1-piperidinyl)-2-isopropyl-2-(4-trifluoromethylphenyl)-)valeronitrile hydrochloride

TLC Rf = 0.77
MS 540 (M+)

Example 150

5-(4-(5H-Dibenzo[a,d]cyclohepten-5-ylidene)-1-piperidinyl)-2-ethyl-2-(3-trifluoromethylphenyl)valeronitrile hydrochloride

TLC Rf = 0.77
MS 526 (M+)

Example 151

2-Butyl-5-(4-(5H-dibenzo[a,d]cyclohepten-5-ylidene)-1-piperidinyl)-2-(3-trifluoromethylphenyl)valeronitrile hydrochloride

TLC Rf = 0.80
MS 554 (M+)

Example 152

5-(4-(5H-Dibenzo[a,d]cyclohepten-5-ylidene)-1-piperidinyl)-2-hexyl-2-(3-trifluoromethylphenyl)valeronitrile hydrochloride

TLC Rf = 0.80
MS 582 (M+)

Test Example 2

As test animals, four male rats (weighing 400 to 440 g) with spontaneous hypertension that were sufficiently adapted for feeding and in which hypertension was confirmed were used.

Physiological saline aqueous solution containing 2.5% Nicolle of sample and 2.5% ethanol was intravenously administered at once in a dose of 1 ml/kg. Blood pressure after the administration was measured by non-blood observation blood pressure measurement method.

The results are shown below.

| Example | Dose (mg/kg) | Degree of Pressure Reduction (mmHg) | |
|---|---|---|---|
| | | 30 minutes | 4 hours |
| 51 | 10 | - 105 | - 10 |
| 52 | 10 | - 130 | - 136 |
| 53 | 10 | - 114 | - 30 |
| 54 | 10 | - 84 | - 16 |
| 56 | 10 | - 37 | 2 |
| 61 | 10 | - 8 | - 7 |
| 66 | 10 | - 61 | - 11 |
| 72 | 10 | - 78 | - 36 |
| 76 | 10 | - 66 | - 14 |
| 78 | 10 | - 14 | 1 |
| 86 | 10 | - 23 | - 9 |
| 94 | 10 | - 121 | - 48 |
| 131 | 10 | - 35 | - 5 |
| 135 | 10 | - 88 | - 19 |
| 136 | 10 | - 126 | - 50 |
| 139 | 10 | - 42 | - 19 |
| 141 | 10 | - 55 | - 7 |
| 142 | 10 | - 90 | - 20 |
| 143 | 10 | - 93 | - 14 |
| 144 | 10 | - 87 | - 19 |

From the foregoing results, it is understood that the ethylamine derivatives of the present invention possess hypotensive activity and are thus useful in providing excellent hypotensives. Accordingly, the present invention is extremely useful, particularly in the pharmaceutical industry.

## Claims

1. An ethylamine derivative of general formula (I) or a pharmaceutically acceptable salt thereof;

$$Q - X - CH_2CH_2 - \overset{\overset{\displaystyle Z}{|}}{N} - CH_2CH_2 - \overset{\overset{\displaystyle W}{|}}{Y} \qquad (I)$$

wherein:
X represents an optionally substituted alkylene bridge, of which one or more carbon atoms may be substituted with an optionally substituted hetero atom and which may include an unsaturated bond;
Y represents any one of the following:

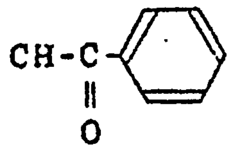
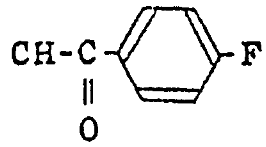
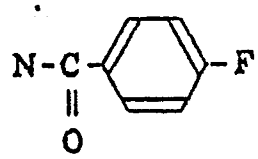

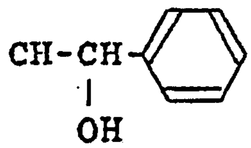
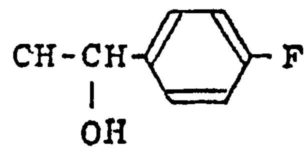

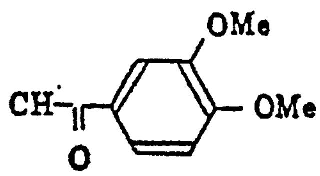
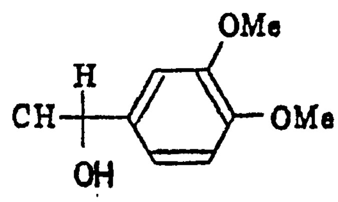

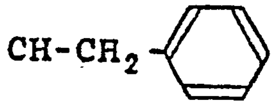

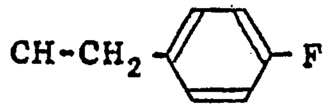

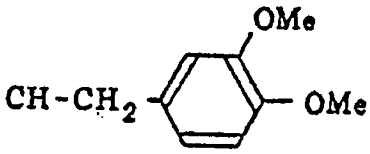

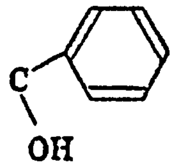

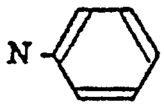

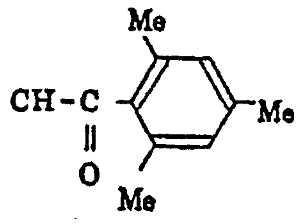

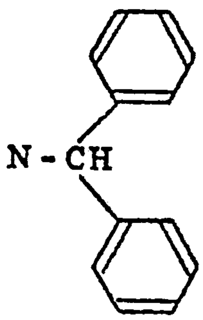

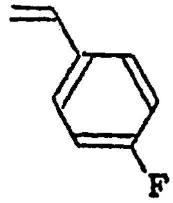

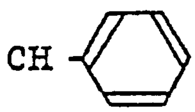

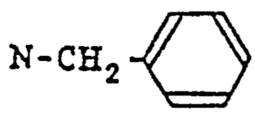

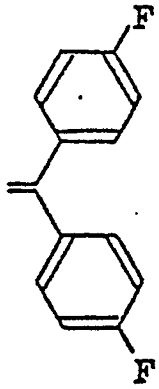

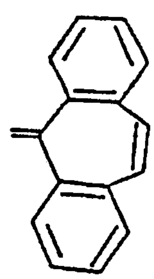

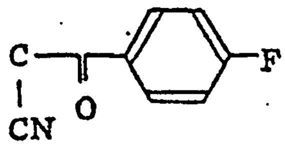
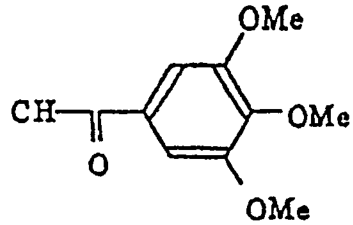

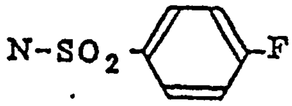
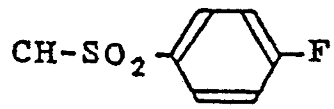

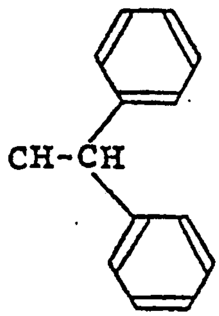
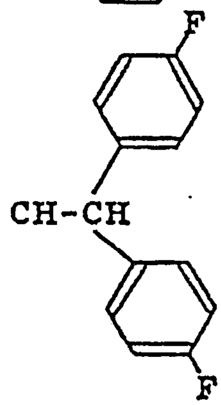

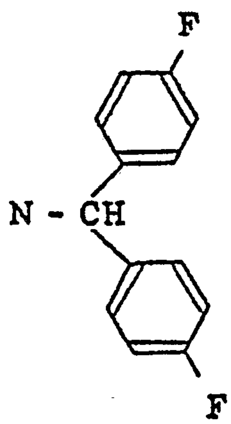
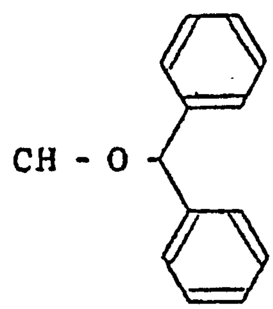

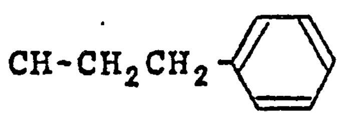
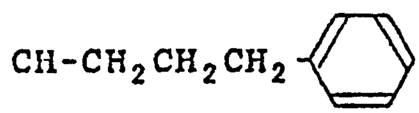

Z represents a methyl group or in combination with W represents an ethylene group ($-CH_2CH_2-$); W represents a hydrogen atom in the case that W is not combined with Z; and Q represents any one of the following:

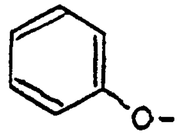

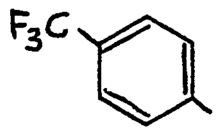

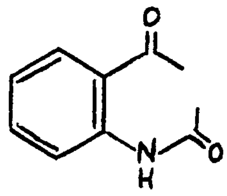

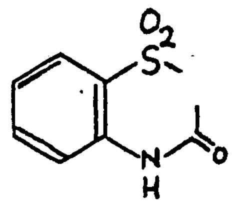

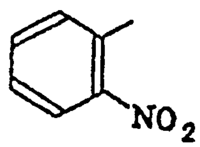

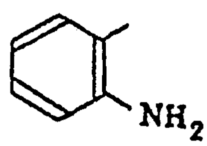

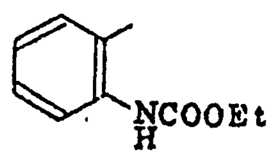

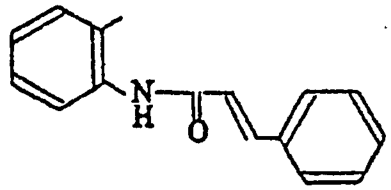

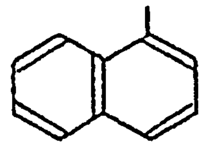

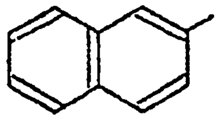

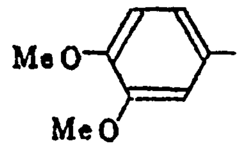

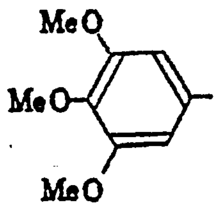

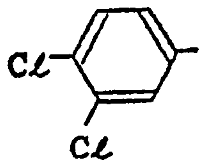

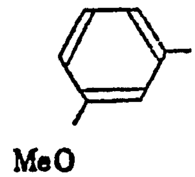

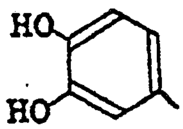

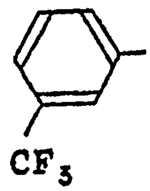

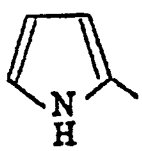

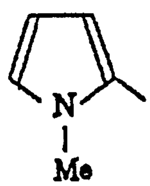

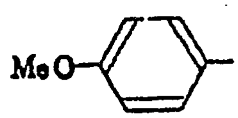

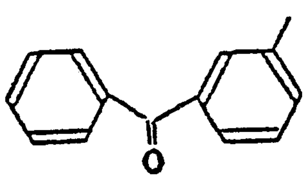

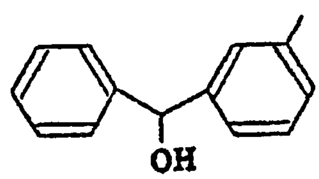

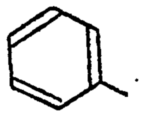

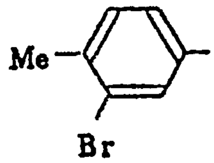

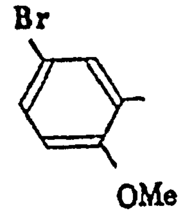

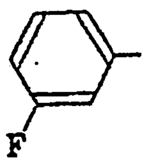

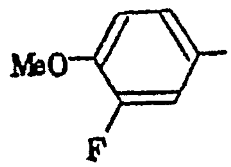

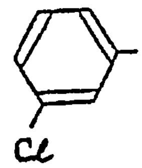

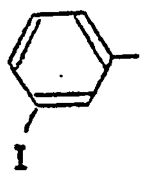

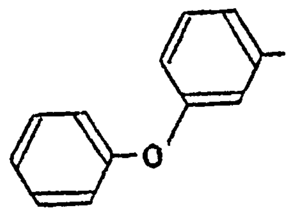

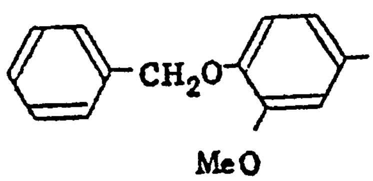

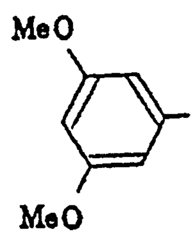

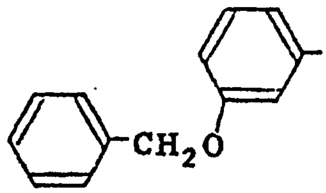

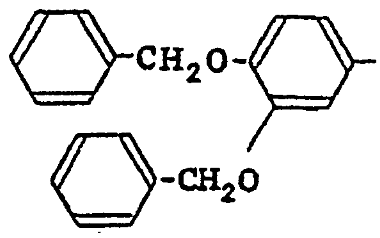

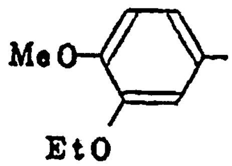

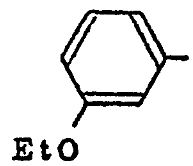

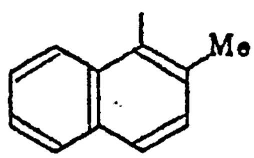

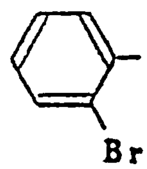

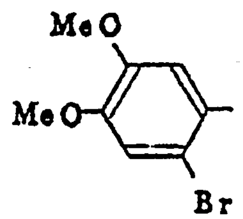

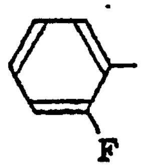

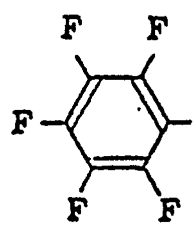

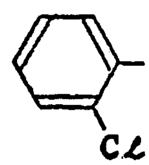

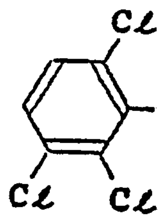
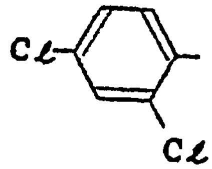
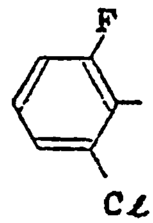

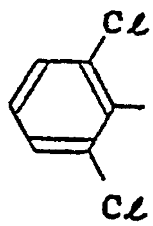
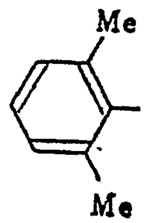
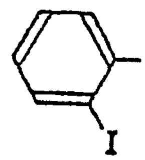

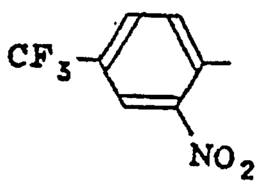
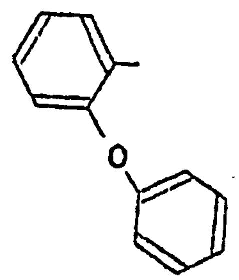

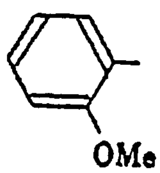
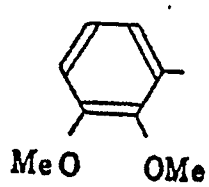
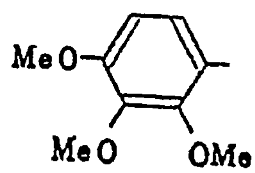

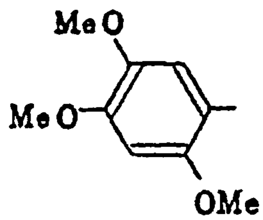

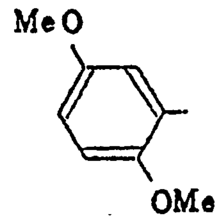

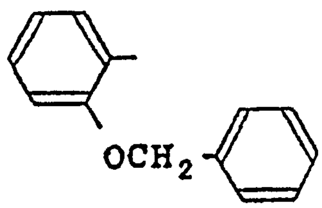

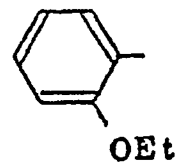

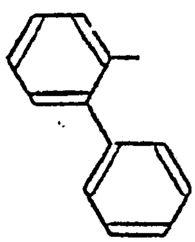

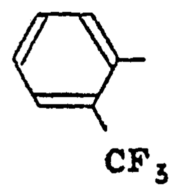

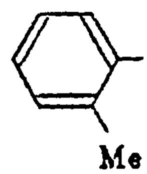

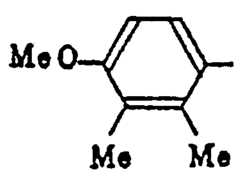

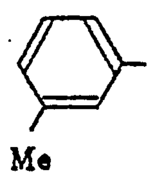

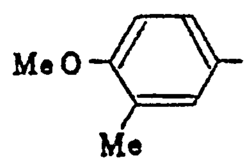

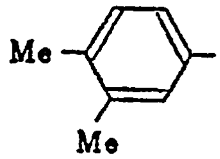

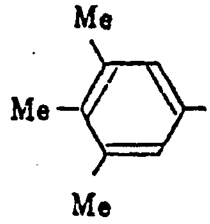

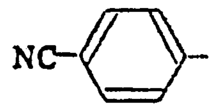

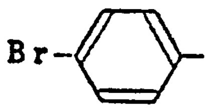

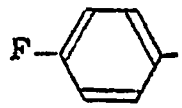

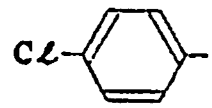

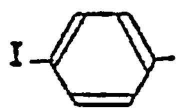

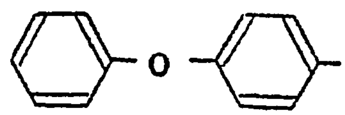

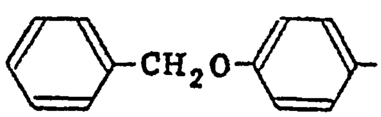

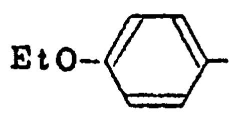

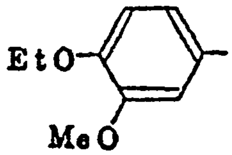

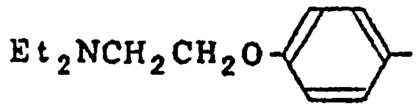

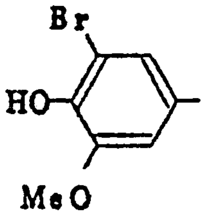

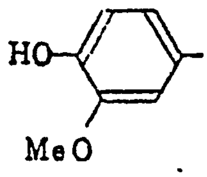

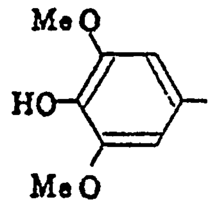

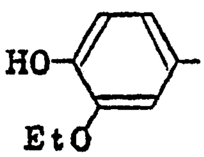

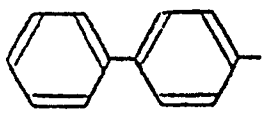

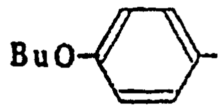

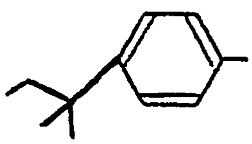

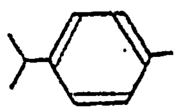

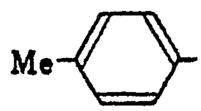

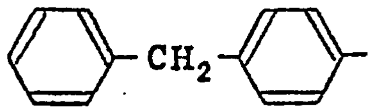
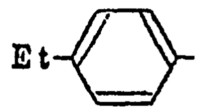
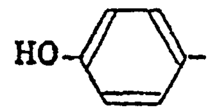

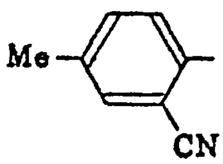
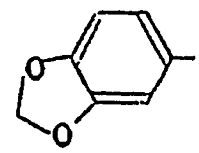
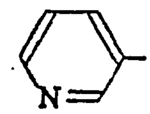

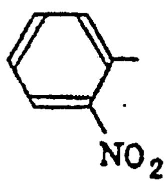
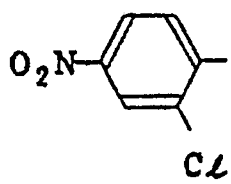
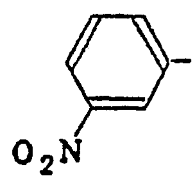

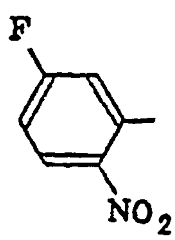
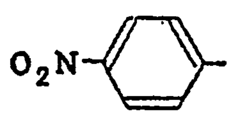
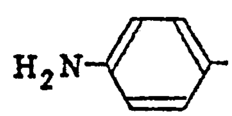

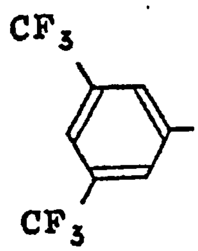
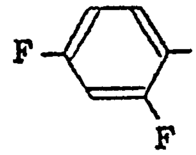
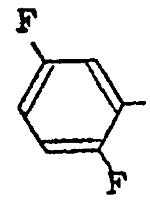

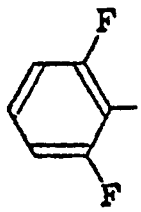
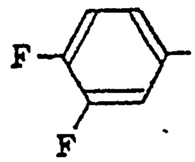
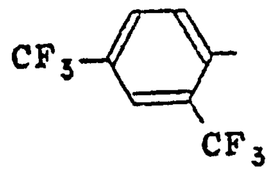

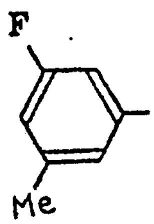
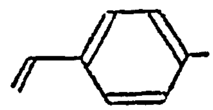
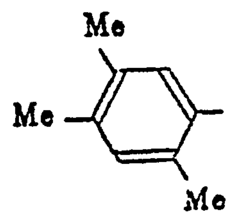

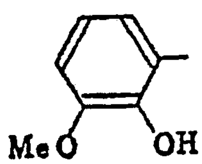
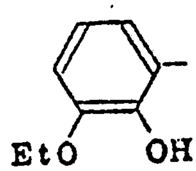
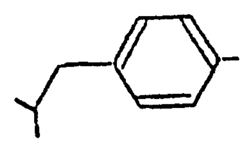

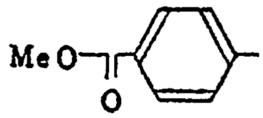
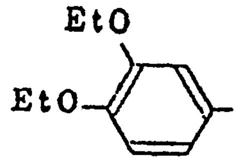
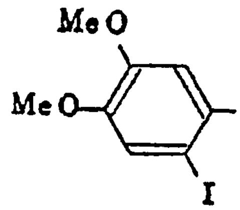

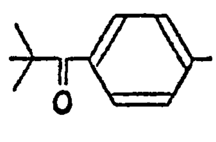
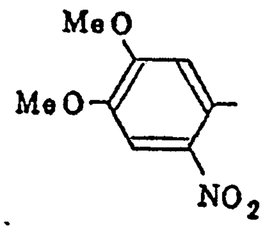

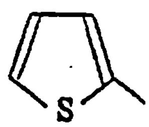
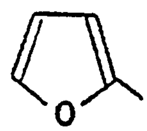
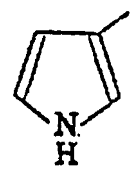

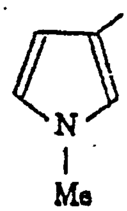
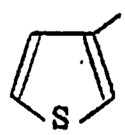
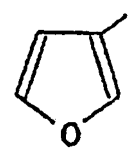

2. An ethylamine derivative or a pharmaceutically acceptable salt thereof according to Claim 1, wherein X in general formula (I) is represented by the following formula (II):

$$-\underset{\underset{B}{|}}{\overset{\overset{A}{|}}{C}} - (CH_2)_n - \qquad (II)$$

wherein n is an integer of 0 to 9; A and B may be the same or different and each represents a hydrogen atom or an optionally substituted alkyl, aralkyl or aryl group and a part of these groups may optionally be substituted with a hetero atom or a hetero atom-containing group.

3. An ethylamine derivative or a pharmaceutically acceptable salt thereof according to Claim 2, wherein when A in general formula (II) is an alkyl group said alkyl group is of 1 to 20 carbon atoms and wherein B represents a hydrogen atom, cyano group, amino group, nitro group, carboxyl group, ester group or

aminocarbonyl group.

4. An ethylamine derivative according to Claim 1, which is any one of the compounds of Examples 2 to 50 or 51 to 152 hereof or a respective free base form thereof.

5. A hypotensive comprising an ethylamine derivative or pharmaceutically acceptable salt thereof according to any one of Claims 1 to 4.

6. A hypotensive according to Claim 5, further comprising at least one of a diuretic, a calcium antagonist, a $\beta$-antagonist, an $\alpha$-antagonist and a convertase inhibitor.

7. A pharmaceutical composition comprising an ethylamine derivative and/or a pharmaceutically acceptable salt thereof according to any one of Claims 1 to 4 and an auxiliary substance, said auxiliary substance being any of a carrier, excipient, binder, preservative, stabilizer, flavour, swelling agent, sweetener, coating agent, buffer, antioxidant.

8. Use of an ethylamine derivative or a pharmaceutically acceptable salt thereof according to any one of Claims 1 to 4 in the preparation of a hypotensive.